# EUROPEAN PATENT APPLICATION

(11) **EP 2 174 578 A1**
(43) Date of publication of application: **14.04.2010**
(21) Application number: 08764144.5
(22) Date of filing: 16.06.2008
(51) Int. Cl.: A61B 1/00, A61B 1/04, A61B 5/07

(54) **IMAGE PROCESSING DEVICE, ITS OPERATING METHOD AND ITS PROGRAM**

(30) Priority: 12.07.2007 JP 2007183582; 15.10.2007 JP 2007268144
(71) Applicant: Olympus Medical Systems Corp., Tokyo 151-0072 (JP)
(72) Inventor: TANIGUCHI, Katsuyoshi, Tokyo 151-0072 (JP); SHIGEMORI, Toshiaki, Tokyo 151-0072 (JP); OGURI, Atsushi, Tokyo 151-0072 (JP); NISHIYAMA, Takeshi, Tokyo 151-0072 (JP)
(74) Representative: Gunzelmann, Rainer
(86) International application number: PCT/JP2008/001549
(87) International publication number: WO 2009/008125

(57) **Abstract**

An image processing apparatus according to the present invention includes: obtainment means for obtaining time-series image data, which is image data captured in time series by a capsule endoscope; image detection means for detecting prescribed image data from the obtained time-series image data; and display control means for displaying a list of the prescribed detection image data among the detected image data.

## Description

### Technical Field

The present invention relates to displaying of observation images captured by a capsule endoscope.

### Background Art

In recent years, capsule endoscope systems have been attracting attention. Capsule endoscope systems can sequentially capture images of organs such as the stomach, small intestine, etc., transmit those captured images to an external receiver for recording the images, and display/reproduce the recorded images on a display device of a computer such as a work station or the like during an observation term starting when a patient swallows, via the mouth, a capsule endoscope having an image capturing device and a wireless device and ending when the capsule endoscope is naturally excreted from the body of the patient (See Patent Document 1 for example).

As described above, in contrast to a normal endoscope, a capsule endoscope continuously captures images of organs for a long time period starting when an examinee swallows the capsule endoscope and ending when the endoscope is naturally excreted, and the observation (or examination) is continued for a long time, e.g., eight hours. Consequently, it has required a very long time and a huge amount of work to confirm all of an immense number of images captured over a long time and to find desired images in a consultation phase, etc. In view of this, a capsule endoscope system having a function of editing examination data obtained over about eight hours by a small-intestine capsule endoscope into a digest of about one or two minutes so as to display the digest as a moving image is disclosed.

However, displaying of a digest as a moving image is not always the best way because displaying of a moving image is performed by displaying still images sequentially in a one-by-one manner, and when, for example, a lesion site is included in the last image, that site cannot be found until a moment immediately before the end of the display, and also, the speed of reproducing the moving image may not be appropriate to the user in some cases.

An observation device of a capsule endoscope has a red-color detection function for detecting a lesion such as bleeding from among at most about 60000 images captured over eight hours. The position of a detected red color is displayed in a red-color detection bar. Doctors have conventionally been required to make the detection position displayed on a red-color detection bar correspond to the position of the reproduced images in a one-by-one manner in order to find images including the detected red color. For example, when all images detected by the red-color detection are to be selected so as to be included in a report, it has been required that such images be selected in a one-by-one manner by pressing a "Frame Step Reproduction" button, and this takes a long time.

In view of the above problem, it is an object of the present invention to realize a listing display of a digest made of a series of images captured by a capsule endoscope or a listing display of images detected in a prescribed image processing.
Patent Document 1
   Japanese Laid-open Patent Publication No. 2007-75158

### Disclosure of Invention

An image processing apparatus according to the present invention includes: obtainment means for obtaining time-series image data, which is image data captured in time series by a capsule endoscope; image detection means for detecting prescribed image data from the obtained time-series image data; and display control means for displaying a list of detection image data, which is the detected image data.

An image processing apparatus according to the present invention includes: obtainment means for obtaining time-series image data, which is image data captured in time series by a capsule endoscope; image detection means for detecting prescribed image data from the obtained time-series image data; display means including a main display area for displaying an image, and two sub-display areas that are smaller than the main display area and that are for displaying an image; display control means for reproducing, in the main display area, detection image data detected from the time-series image data by the image detection means, reproducing and displaying detection image data before and after the detection image data in each of the sub-display areas, displaying, when the reproduction is stopped, detection image data at the moment of the stoppage in the main display area, and displaying the time-series image data corresponding to frame images before and after the detection image data in the sub-display areas.

A method of operating an image processing apparatus according to the present invention includes: obtaining time-series image data, which is image data captured in time series by a capsule endoscope; detecting prescribed image data from the obtained time-series image data; and displaying a list of detection image data, which is the detected image data.

A method of operating an image processing apparatus according to the present invention includes: obtaining time-series image data, which is image data captured in time series by a capsule endoscope; detecting prescribed image data from the obtained time-series image data; displaying a main display area for displaying an image, and two sub-display areas that are smaller than the main display area and that are for displaying an image; reproducing, in the main display area, detection image data detected from the time-series image data, reproducing and displaying detection image data before and after the detection image data in each of the sub-display areas, displaying, when the reproduction is stopped, detection image data at the moment of the stoppage in the main display area, and displaying the time-series image data corresponding to frame images before and after the detection image data in the sub-display areas.

An image display program for making a computer perform a process of displaying an image according to the present invention makes the computer execute: an obtainment step of obtaining time-series image data, which is image data captured in time series by a capsule endoscope; an image detection step of detecting prescribed image data from the obtained time-series image data; and a display control step of displaying a list of detection image data, which is the detected image data.

### Brief Description of Drawings

Fig. 1 shows a capsule endoscope used for in-body-cavity examinations according to the first embodiment and peripheral devices for the capsule endoscope;
Fig. 2 schematically shows an internal configuration of a work station 7 for performing image processing on the data of images captured by a capsule endoscope 1 according to the first embodiment;
Fig. 3 shows an observation screen of an endoscope system according to the first embodiment;
Fig. 4 shows a screen displayed when a listing display is selected as a display method for a digest display according to the first embodiment;
Fig. 5 is a view showing an example of a case where "Reproduce around This Frame" 54f is selected as a command according to the first embedment (first);
Fig. 6 is a view showing an example of a case where "Reproduce around This Frame" 54f is selected as a command according to the first embedment (second);
Fig. 7 shows a screen displayed when a moving image display is selected as a display method for a digest display according to the first embodiment;
Fig. 8 shows a listing display according to the second embodiment;
Fig. 9 shows assignment of comments to images in a displayed list according to the second embodiment;
Fig. 10 shows a button for outputting, en masse and to a report, images in a displayed list according to the second embodiment;
Fig. 11 shows a display screen that displays a list and a color bar/time bar at the same time so as to indicate the position of an image selected on the list according to the third embodiment (example 1);
Fig. 12 shows a list display window and an abstract information display window, displaying at the same time a list and the color bar/time bar on different screens so as to indicate the position of an image selected on the list, according to the third embodiment (example 2);
Fig. 13 shows a list display screen that displays a section of a listed image on the color bar/time bar according to the fourth embodiment (example 1);
Fig. 14 shows a list display screen that displays a list-displayed section on the color bar/time bar according to the fourth embodiment (example 2);
Fig. 15 shows a list display screen that displays a list-displayed section on the color bar/time bar according to the fourth embodiment (example 3);
Fig. 16 shows a list display screen that displays a list-displayed section on the color bar/time bar according to the fourth embodiment (example 4);
Fig. 17 shows a list display screen that scrolls the list in arbitrary units according to the fifth embodiment;
Fig. 18 shows a transition from a moving-image display screen to a list display screen according to the sixth embodiment (example 1);
Fig. 19 shows a transition from a moving-image display screen to a list display screen according to the sixth embodiment (example 2);
Fig. 20 shows a transition from a moving-image display screen 31 to a list display screen 100 according to the sixth embodiment (example 3);
Fig. 21 shows a transition from the moving-image display screen 31 to the list display screen 100 according to the sixth embodiment (example 4);
Fig. 22 shows a transition from the moving-image display screen 31 to the list display screen 100 according to the sixth embodiment (example 5);
Fig. 23 shows a transition from a list display screen to a moving-image display screen according to the seventh embodiment (example 1);
Fig. 24 shows a transition from a list display screen to a moving-image display screen according to the seventh embodiment (example 2);
Fig. 25 shows a transition from a list display screen to a moving-image display screen according to the seventh embodiment (example 3);
Fig. 26 shows a list display screen that displays a page section of a listed image on the color bar/time bar according to the eighth embodiment;
Fig. 27 shows a list display screen on which a check mark can be placed on an image that attracts user's attention on a list display screen according to the ninth embodiment (example 1);
Fig. 28 shows a list display screen on which a check mark can be placed on an image that attracts user's attention on a list display screen according to the ninth embodiment (example 2);
Fig. 29 shows changing of a display state of an interesting image in a sub-display area for displaying a list according to the tenth embodiment (example 1);
Fig. 30 shows changing of a display state of an interesting image in a sub-display area for displaying a list according to the tenth embodiment (example 2);
Fig. 31 shows an addition of an image selected on the moving-image display screen to a list display screen according to the eleventh embodiment;
Fig. 32 shows a list display screen on which marks of images extracted for a listing display are placed on the color/time bar according to the twelfth embodiment;
Fig. 33 shows an example of a list display screen for displaying a desired range on the color bar/time bar having marks at positions corresponding to images extracted for a listing display according to the twelfth embodiment (variation example 1);
Fig. 34 shows a list display screen according to the twelfth embodiment (variation example 2);
Fig. 35 shows the list display screen 100 according to the thirteenth embodiment;
Fig. 36 shows an enlarged display performed when a mouse cursor 161 is moved to an image displayed in a list display area 101 according to the fourteenth embodiment;
Fig. 37 shows a detection condition setting screen for setting a condition for detecting a digest image according to the fifteenth embodiment;
Fig. 38 shows the list display screen 100 in which an image group in a particular section is specified in a time-series image group and time-series images in the specified range are displayed, according to the sixteenth embodiment;
Fig. 39 shows a detection condition setting screen for detecting a digest image for each organ (site) or each particular section according to the seventeenth embodiment;
Fig. 40 shows a sequential turning of pages in a listing display performed after a time period set by a user has elapsed in a case when a listing display covers plural pages according to the eighteenth embedment;
Fig. 41 shows a detection condition setting screen 200 for setting an observation time period according to the nineteenth embodiment; and
Fig. 42 shows an observation screen for an endoscope system according to the twentieth embodiment.

### Best Mode of Carrying Out the Invention

### <First Embodiment>

In the present embodiment, explanations will be given for a listing display of a digest of a series of images captured by a capsule endoscope. Use of the present embodiment enables a listing display of a digest of a series of images captured by a capsule endoscope or images detected in prescribed image processing, and thereby necessary images can be determined efficiently.

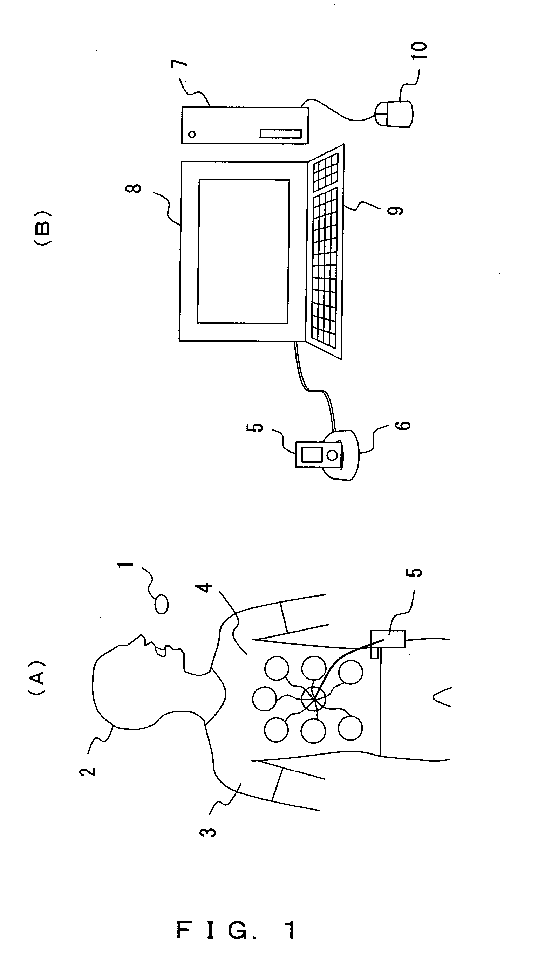

Fig. 1 shows a capsule endoscope used for in-body-cavity examinations in the present embodiment and peripheral devices for the capsule endoscope. As shown in view (A), the examination system using a capsule endoscope 1 includes the capsule endoscope 1, antenna pads 4, and an external device 5.

The capsule endoscope 1 is swallowed by a patient 2 via the mouth so that it can perform examinations in the body cavity. The external device 5 is a receiver that is provided out of the body of the patient 2 and is connected to a plurality of antenna pads 4 for wirelessly receiving data of images captured by the capsule endoscope 1.

The external device 5 has a portable storage medium such as a compact flash (registered trademark) memory card so that the image information transmitted from the capsule endoscope 1 and received by the external device 5 is recorded during an examination in a body cavity.

The above image information is retrieved by a terminal device 7 such as a personal computer, a work station, or the like (the present embodiment uses the work station 7) via the portable storage medium.

The external device 5 is mounted on a cradle 6 as shown in Fig. 1(B) or uses a USB cable or the like (not shown) so that it is electrically connected to the work station 7 in order to transfer information to the work station 7. Thereby, the work station 7 can retrieve image data stored in the portable storage medium inserted into the external device 5. It is also possible to connect the reading device for the portable storage medium to the work station 7 and insert the portable storage medium to the reading device so that image data stored in the portable storage medium is read out and is retrieved by the work station 7.

The work station 7 retrieves images on the basis of operations on an input/manipulation device such as a keyboard 9, a mouse 10, or the like. Upon doing this, it is also possible to detect an electrical connection of a USB or the like so as to retrieve images on the basis of the detection. The images retrieved by the work station 7 can be displayed on a display device 8 or be output to a printer.

As shown in Fig. 1(A), when the patient 2 swallows the capsule endoscope 1 to receive an endoscope examination, the plurality of antenna pads 4 are attached to a jacket 3 that the patient 2 wears. In such a case, the antenna pads 4 can also be attached to the patient' s body directly. The data of images captured by the capsule endoscope 1 is wirelessly transmitted to the antenna pads 4 so that the antenna pads 4 can receive the image data. Then, the image data is stored in the external device 5 connected to the antenna pads 4. The external device 5 is attached to the belt of the patient 2 by means of, for example, a detachable hook.

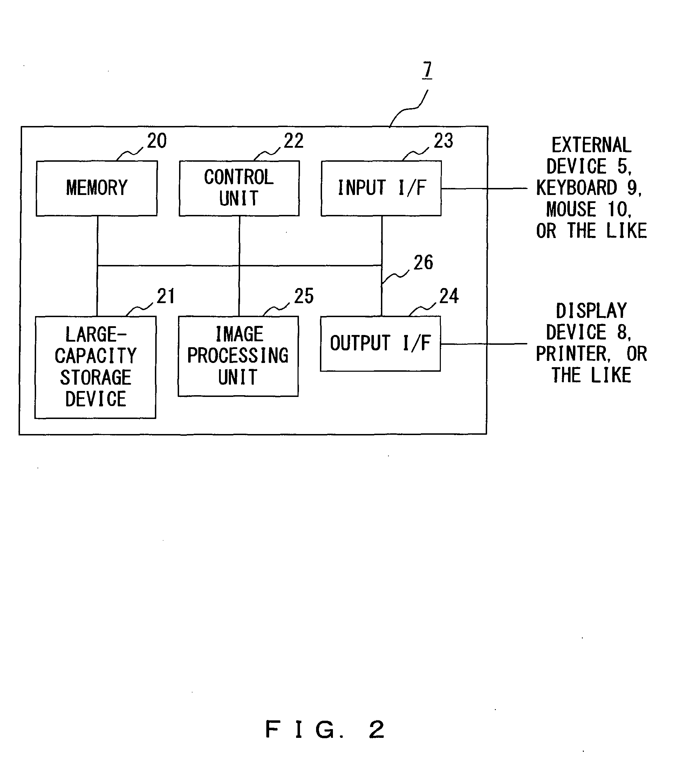

Fig. 2 schematically shows an internal configuration of the work station 7 for performing image processing on the data of images captured by the capsule endoscope 1 in the present embodiment. The work station 7 includes memory 20, a large-capacity storage device 21, a control unit 22, an input I/F 23, an output I/F 24, an image processing unit 25, and a bus 26 for connecting them.

The memory 20 is memory such as RAM (Random Access Memory), ROM (Read Only Memory), or the like that is used for various processes. The large-capacity storage device 21 is a hard disk drive (HDD) for storing a large amount of data.

The input I/F 23 is an input interface for retrieving image data from the external device 5 (or a transportable storage medium such as a compact flash (registered trademark) memory card or the like) and inputs data or instructions given from the manipulation unit such as the keyboard 9, the mouse 10, or the like. The output I/F 24 is an output interface for outputting images captured by the capsule endoscope to the display device 8 and outputting data to a printer or the like.

The control unit 22 is implemented by a CPU or the like that executes various processing programs stored in the large-capacity storage device 21. The control unit 22 also controls screens for performing a listing display or a moving image display, or various types of processing in the above embodiment of the present invention.

An image processing unit 25 obtains image data from the external device 5 or the large-capacity storage device 21 on the basis of control performed by the control unit 22, and performs, on the obtained image data, various image processes such as concentration conversion (gamma conversion or the like), smoothing (noise elimination or the like), sharpening (edge reinforcement or the like), image recognition (detection of characteristic image region, calculation of average color, or the like) or the like.

The data of images captured by the capsule endoscope 1 is transmitted to the external device 5 in a point-by-point manner, and is stored in the portable storage medium in the external device 5. The stored image data is stored in the large-capacity storage device 21 in the work station 7 via the external device 5 mounted on the cradle 6 or via the portable storage medium set on the reading device to be electrically connected to the work station 7. Thereby, the images captured by the capsule endoscope 1 are retrieved by the work station 7.

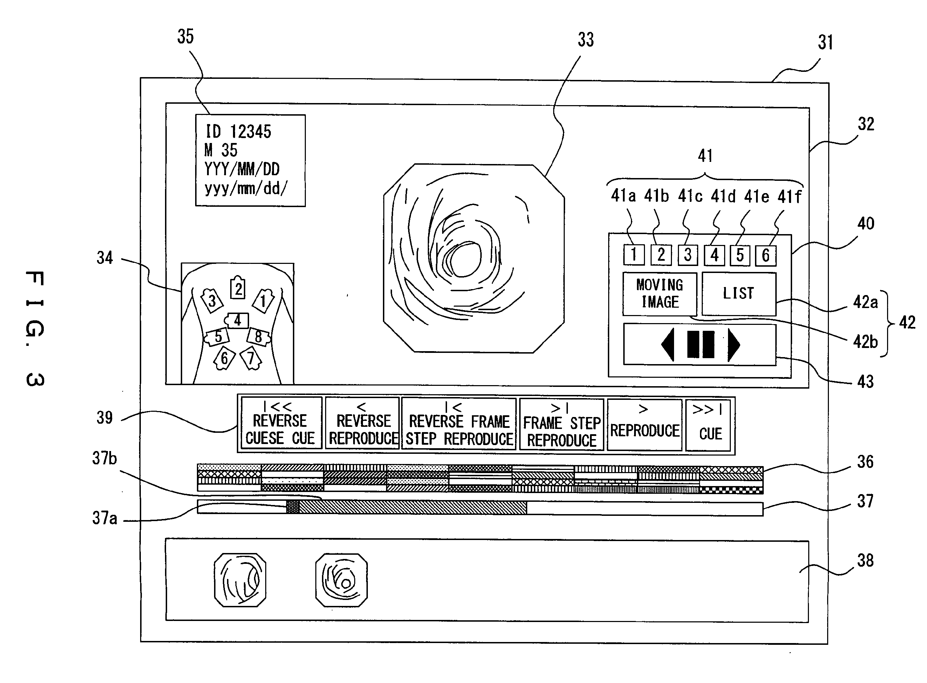

Fig. 3 shows an observation screen of an endoscope system in the present invention. As shown in Fig. 3, the display device 8 displays a window 31 serving as a GUI screen ("Consultation/Diagnosis" window). In the window 31, a main display area 32 for displaying a main display image or the like, an image manipulation area 39 for displaying various image manipulation buttons in the form of icons, a color bar 36 and a time bar 37 serving as a time scale representing the term of capturing images in the body cavity of a patient, a sub-display area 38 for displaying a thumbnail image or the like are parallelly displayed in this order from the top to the bottom of the display screen.

In the main display area 32, a main display image 33 that is an image selected from among examinee body cavity images, an antenna arrangement chart 34 for schematically showing the arrangement of the antennas 11 on the patient 2, a patient information display box 35, and a digest display manipulation area 40 are displayed together with instruction information input through a manipulation unit such as the keyboard 9, the mouse 10, or the like.

The patient information display box 35 displays, as text information, the name of the patient 2, the ID number, the sex, the age, the birth date, the data of the image capturing, the clock time at which the image was captured, and the like in an associated state with the examinee body cavity image selected as the main display image 33. The main display area 32 can display two or more prescribed main display images in accordance with prescribed instructions.

The antenna arrangement chart 34 schematically displays an arrangement of the antenna pads 4 together with the partial outline of the body of the patient 2. Also, the antenna arrangement chart 34 displays, near the antennas 11, the antenna numbers of the respective antennas as the identification numbers.

The digest display manipulation area 40 displays digest display algorithm selection switches 41 (41a, 41b, 41c, 41d, and 41e), digest display method selection switches 42 (a "List" switch 42a and a "Moving Image" switch 42b), and a moving-image control key 43.

The digest display algorithm selection switches 41 are switches for specifying an algorithm for selecting images to be displayed as a digest (referred to as digest images hereinafter) when, for example, several hundred through several thousand images are to be extracted from among an immense number of time-series images (case data) captured by the capsule endoscope and are to be displayed as a digest.

When switch 1 (41a) is pressed, images are extracted at prescribed intervals from among an immense number of images captured by the capsule endoscope so that, for example, the 20th image, the 40th image, the 60th image...... are extracted to be displayed as a digest.

When switch 2 (41b) is pressed, when, for example, the n-th image and the (n+1)th image are similar to each other, the n-th image is deleted, and this process is performed for all the images, and only images involving large changes (movement) with respect to the other images remain, so that such images are displayed as a digest. Whether or not an image is similar to other images can be determined on the basis of, for example, whether or not the difference in pixel value between the two images is higher than a threshold value.

In addition, combinations of compared images are not limited to the combination of the n-th image and the (n+1)th image, and the n-th image and the (n±m) th image can be compared, where m is an arbitrary integer. It is also possible to extract image data on the basis of a difference in image data between the n-th image and the average value of the p images (where n and p are arbitrary integers) before and after the n-th image.

When switch 3 (41c) is pressed, only images involving characteristic sites that seem to be bleeding or a lesion are extracted to be displayed as a digest. As an example of a method of extracting images involving bleeding areas, images including red-color components in excess of a prescribed threshold value in a local image region may be extracted. As an example of a method of extracting images involving lesion sites, images including a pattern that corresponds to or corresponds to within a prescribed range of allowable error, patterns such as colors of a lesion site or a lesion shape that are registered in advance may be extracted.

When switch 4 (41d) is pressed, images unnecessary to diagnoses such as images including only organs other than the organ to be observed or images including only residue are not extracted, and only the remaining images are extracted to be displayed as a digest. As an example of extracting images including organs to be observed, images may be extracted on the basis of similarity of characteristics (such as color components) of images of the target organ because organs can be identified on the basis of the average of the color components in an entire image.

When switch 5 (41e) is pressed, digest images based on the combination of the functions of switch 2 (41b), switch 3 (41c), and switch 4 (41d) can be displayed. In other words, images involving movement, lesions, and a red color can only be extracted to be displayed as a digest.

When switch 6 (41f) is pressed, one of the functions of switches 1 (41a) through 5 (41e) can be applied for about eight hours only to an organ portion (such as a small intestine portion) selected by a user in advance from among the case data.

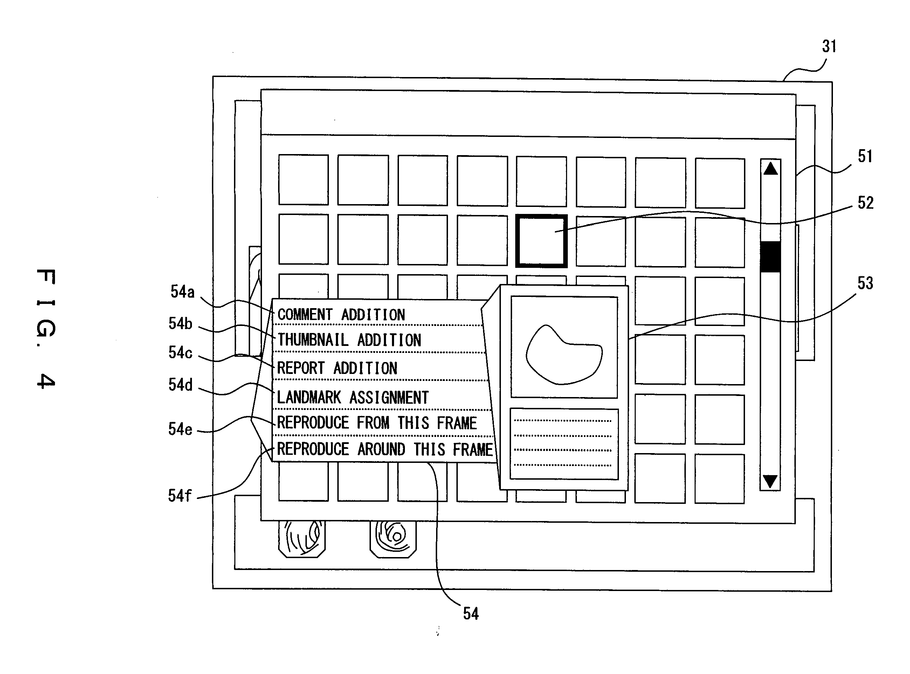

When the "List" switch 42a is pressed, digest images can be displayed in a listed manner. While digest images are displayed, the moving-image control key 43 is made to enter a hidden state or a non-active state. The listing display of digest images will be described in detail when Fig. 4 is explained.

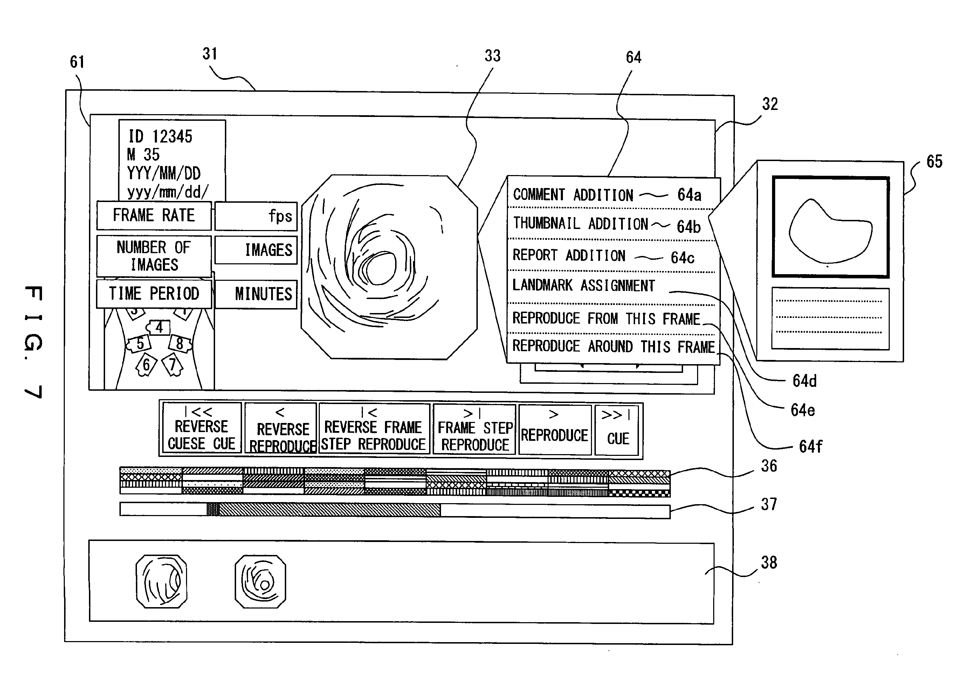

When the "Moving Image" switch 42b is pressed, digest images are displayed sequentially as a moving image. When doing this, while the moving-image control key 43 is in a hidden state or a non-active state, the "Moving Image" control key 43 is displayed in a usable state. The displaying of digest images as a moving image will be described in detail when Fig. 7 is explained.

The moving-image control key 43 is a switch for performing controls such as reproduce, reverse reproduce, and pause when digest images are displayed as a moving image.
The color bar 36 displays, in time series, the average colors of the respective images included in a series of examinee body cavity images. Specifically, each display area corresponding to a clock time on the color bar 36 displays the average color of the examinee body cavity image captured at that clock time. A series of examinee body cavity images have average colors peculiar to the organs that have been captured, and accordingly observers or others can easily determine what organs are included in the respective examinee body cavity images captured at the respective clock times, on the basis of the transition of the average colors along the time axis (horizontal axis in Fig. 3) of the color bar 36.

On the time bar 37, a slider 37a that can be moved along the time axis on the time bar 37 is displayed. The slider 37a specifies a clock time at which the examinee body cavity image displayed as the main display image 33 was captured, and can move along the time bar 37 in association with the switching of the main display image 33 to be displayed.

The sub-display area 38 displays thumbnail images selected and extracted from among a series of examinee body cavity images. Specifically, in response to prescribed button manipulations or mouse manipulations, etc., the examinee body cavity image that is being displayed as the main display image 33 at the moment of that manipulation is added as a thumbnail image and displayed on the sub-display area 38.

Fig. 4 shows a screen displayed when a listing display is selected as a display method for digest display according to the present embodiment. As described above, a digest list display screen 51 can be displayed when the "List" switch 42a is pressed. The images to be displayed in the list are thumbnail images. The user can arbitrarily select the number of the images to be displayed in the list, such as 7 (vertical) x 7 (horizontal), 10 (vertical) x 10 (horizontal), etc. The size of the thumbnail images can also be selected arbitrarily by the user. It is also possible to automatically display a number of images corresponding to a specified size of the thumbnail images.

When the digest images are displayed in a listed manner, a frame image including a characteristic site that seems to be bleeding or a lesion is displayed in an emphasized manner by, for example, being highlighted by a frame denoted by numeral 52 or is displayed with prescribed identification information. Thereby, such images can be noticeable to users.

When an arbitrary image is selected from among the listed digest images, a pop-up menu 54 displaying plural commands is displayed. The commands displayed on the pop-up menu 54 include "Comment Addition" 54a, "Thumbnail Addition" 54b, "Report Addition" 54c, "Landmark Assignment" 54d, "Reproduce from This Frame" 54e, and "Reproduce around This Frame" 54f.

When "Comment Addition" 54a is selected, a comment input screen 53 is displayed so that remarks on the frame image on the displayed list can be added as a comment. It is also possible to automatically add, to a report, a frame image to which a comment has been added.

When "Thumbnail Addition" 54b is selected, the thumbnail image of the selected frame image can be registered (thumbnail register) in the sub-display area 38. It is also possible to permit the addition of a thumbnail image when the selected frame image is double-clicked or right-clicked.

When "Report Addition" 54c is selected, the selected frame image can be directly pasted to a report that describes the remarks, diagnosis, or the like on the case.
When "Landmark Assignment" 54d is selected, a landmark can be assigned to the selected frame image. When a landmark is assigned, a prescribed mark is displayed at the position, along the time bar 37, corresponding in time to the image that has been assigned the landmark.

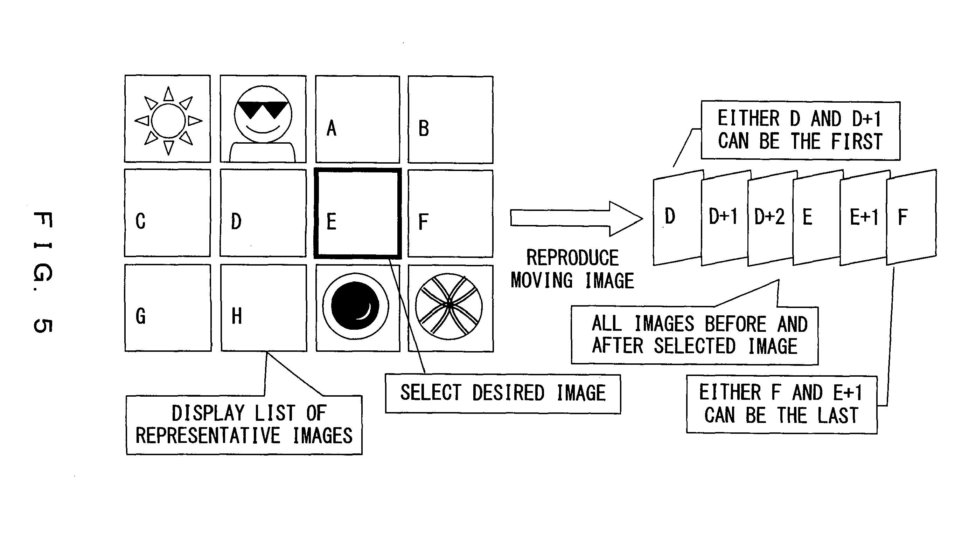

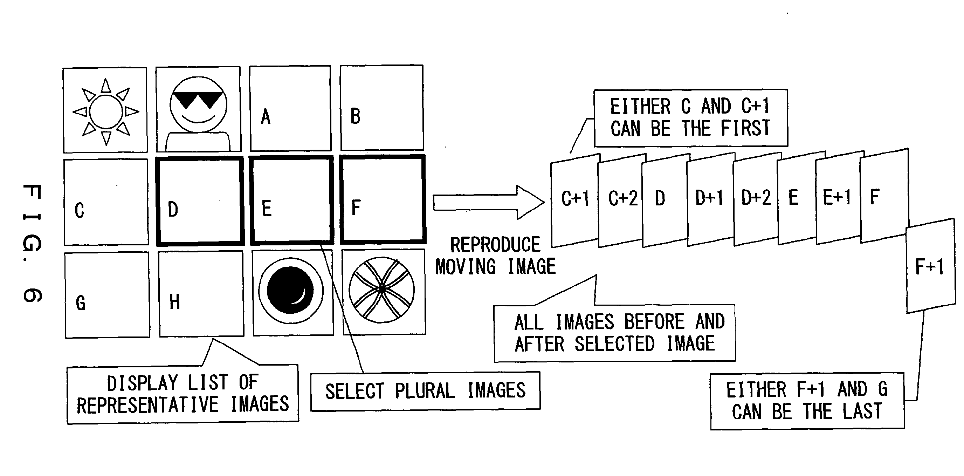

When "Reproduce from This Frame" 54e is selected, the window transitions to the window 31 so that a normal moving image after the selected frame image is reproduced on the main display area 32.
When "Reproduce around This Frame" 54f is selected, the current window transitions to the window 31 so that the x (x is an arbitrary integer) images before and after the selected frame images are sequentially displayed in a one-by-one manner in the main display area 32. The number (x) of the images may be set using the "Setting" on the menu bar in advance, or may be set by selecting "Reproduce around This Frame" 54f on a pop-up menu displayed in response to double-clicking and setting a setting command in a new pop-up menu that has been additionally displayed. When the "Reproduce around This Frame" 54f is selected, the window transitions to the window 31, and all images between the selected frame image and the image extracted immediately before the selected frame image or all images between the selected frame image and the image extracted immediately after the selected image are sequentially displayed in a one-by-one manner on the main display area 32, as shown in Figs. 5 and 6.

Fig. 7 shows a screen displayed when a moving image display is selected as a display method for a digest display according to the present embodiment. When the "Moving Image" switch 42b is pressed, digest images are displayed sequentially in a one-by-one manner as a moving image in the main display area 32.

On the left side of the screen, a "Frame Rate" setting box 61, a "Number of Images" setting box 62, and a "Time" display box 63 are displayed. In the "Frame Rate" setting box 61, a frame rate can be set for sequentially displaying images extracted in accordance with the digest display algorithm selection switch 41 as a moving image. In the "Number of Images" setting box 62, a number of the images to be displayed sequentially as a moving image can be set when images extracted in accordance with the digest display algorithm selection switch 41 are to be displayed. The "Time" display box 63 displays a time period of reproducing a moving image based on the frame rate and the number of images respectively set in the "Frame Rate" setting box 61 and the "Number of Images" setting box 62.

When digest images are displayed as a moving image, frame images including characteristic sites that seem to be bleeding or to be a lesion are emphasized by means of, for example, highlighting, or are displayed with prescribed identification information. Thereby, such images are noticeable to users.

A pause button on the moving-image control key 43 is pressed while a moving image is being reproduced so that the moving image is paused with an arbitrary frame image being displayed. When this paused image is right-clicked or double-clicked, a pop-up menu 64 displaying plural commands is displayed. The commands displayed on a pop-up menu 64 include "Comment Addition" 64a, "Thumbnail Addition" 64b, "Report Addition" 64c, "Landmark Assignment" 64d, "Reproduce from This Frame" 64e, and "Reproduce around This Frame" 64f.

When "Comment Addition" 64a is selected, a comment input screen 63 is displayed so that comments can be added to the selected frame image on the list. It is also possible to automatically add, to a report, a frame image to which a comment has been added.

When "Thumbnail Addition" 64b is selected, the thumbnail image of the selected frame image can be registered (thumbnail register) in the sub-display area 38. It is also possible to permit addition of a thumbnail image when the selected frame image is double-clicked or right-clicked.

When "Report Addition" 64c is selected, the selected frame image can be directly pasted to a report.
When "Landmark Assignment" 64d is selected, a landmark can be assigned to the selected frame image. When a landmark is assigned, a prescribed mark is displayed at the position, on the time bar 37, corresponding in time to the image that has been assigned the landmark.

When "Reproduce from This Frame" 64e is selected, a normal moving image after the selected frame image is reproduced.
When "Reproduce around This Frame" 64f is selected, the x (x is an arbitrary integer) images before and after the selected frame image are sequentially displayed in a one-by-one manner. The number (x) of the images may be set using "Setting" on the menu bar in advance, or may be set by selecting "Reproduce around This Frame" 64f on a pop-up menu displayed in response to double-clicking and setting a setting command in a new pop-up menu that has been additionally displayed.

In addition, the commands are displayed on a pop-up menu in the present embodiment. However, this example is not intended to limit the scope of the present invention, and they may be displayed on, for example, a pull-down menu or buttons that are displayed in advance.

Also, in the present embodiment, images extracted by a digest display algorithm are displayed in a listed manner. However, this example is not intended to limit the scope of the present invention, and images other than such images may be displayed in a listed manner.

According to the present embodiment, an algorithm for performing digest display can be selected in accordance with the purpose of a user. Also, plural digest display methods may also be provided so that users can select a desired method. Also, observation methods mainly for digest display can be provided so that the observation time period can be reduced.

### <Second Embodiment>

In the first embodiment, digest images are displayed in a listed manner. However, in the present embodiment, at least one image process routine is performed for case data obtained by a capsule endoscope, and images detected in each image process are listed and displayed with the names of the assigned image processing routines as labels. The work station 7 that is used in the first embodiment is also used in the present embodiment, and accordingly the explanation thereof is omitted.

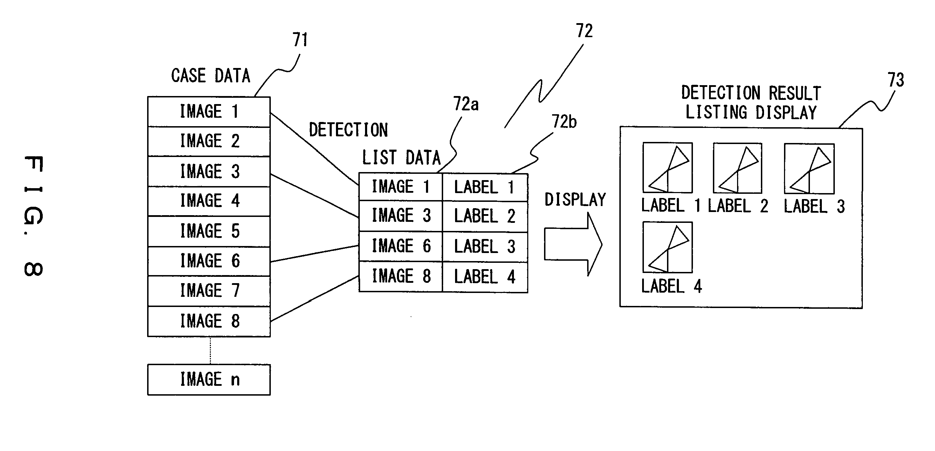

Fig. 8 shows a listing display according to the present embodiment. First, case data 71 consisting of about 60,000 images captured by a capsule endoscope is transferred from the external device 5. The image processing unit 25 performs at least one image processing routine, for the case data 71, such as color detection (red-color detection, white-color detection, etc.) for detecting images whose luminance value based on a prescribed color component is higher than a prescribed threshold value, or polyp detection for detecting polyps on the basis of patterns such as shapes, colors, and the like of polyps that are registered in advance.

Next, the control unit 22 generates list data 72 including only images detected by the image processing from among the case data 71. In the pieces of the list data 72, the images detected in the image processing and the label names are associated with each other. Label names are the names of the image detection routines (such as red-color detection, white-color detection, etc.) used in the above image processing.

Next, the control unit 22 displays, in a listed manner, the images of the case data as a detection result listing display 73 on the display device 8 on the basis of the list data 72. In this displaying, the label names are displayed near the listed images. Additionally, not only text but also marks such as identification figures or the like can be displayed as label names.

This makes it possible to see a list of the images detected by a prescribed image processing routine, and accordingly a time period consumed for observing detection results can be reduced.
Next, explanations will be given for assignment of comments to the listed images.

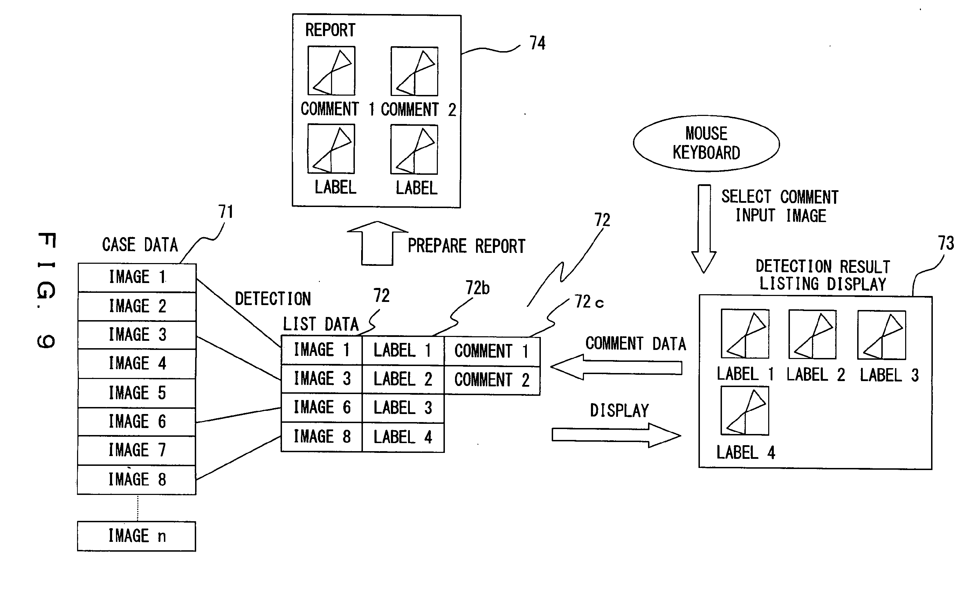

Fig. 9 shows assignment of comments to listed images according to the present embodiment. One of images displayed on the detection result listing display 73 is selected by using an input device such as a mouse, keyboard, or the like so that comments for that image can be input.

Then, the input comment data 72c is assigned to its corresponding image data in the list data 72.
Further, when an image is to be included in a report by means of an input device such as a mouse or the like, that comment is automatically included in the report 74.

Thereby, comments can easily be added to images detected by a red-color detection function, a white-color detection function, or the like so that diagnoses can be made efficiently. Also, an image to be included in a report can easily be selected from among detected images so that reports can be prepared efficiently.

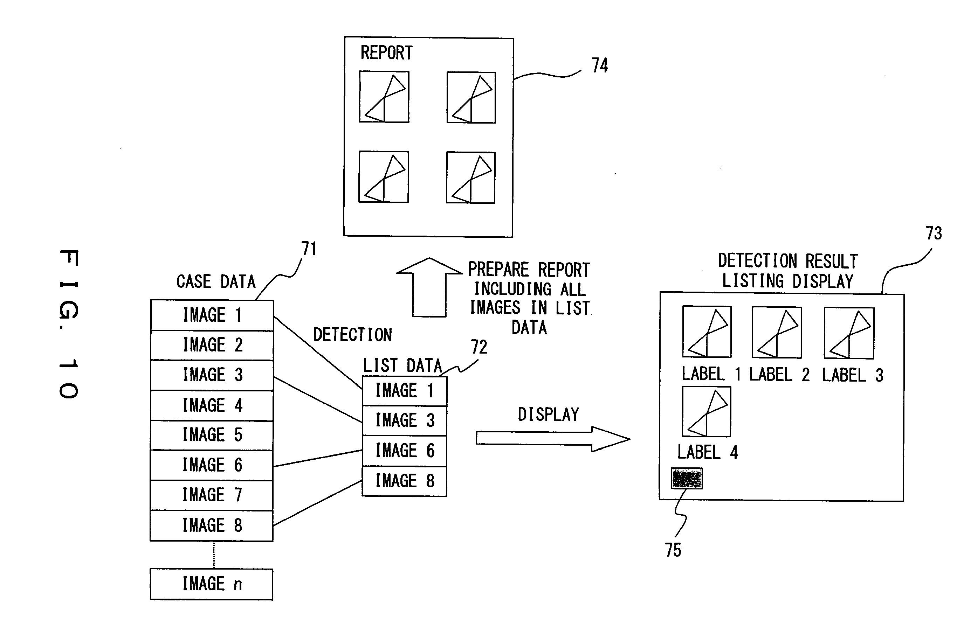

As shown in Fig. 10, it is also possible to include, en masse, the images of the list data 72 in a report when a prescribed button 75 is pressed on the screen for the detection result listing display 73. This makes it possible to prepare a report including all the detected images so that reports can be prepared efficiently.

According to the present embodiment, list data including all detected images is generated, and the images based on that list data are displayed on a screen so that detection results can be viewed quickly. Also, comments that can be used in reports or the like can be input, by means of an input device such as a keyboard or the like, to the images displayed in a list of the detection results. Further, by pressing a prescribed button set on the screen, all images detected can automatically be output to reports.

### <Third Embodiment>

In the present embodiment, explanations will be given for a configuration in which a listing display based on thumbnail images and an abstract information display covering all images (a color bar, a time bar, and the like) are performed on the same screen, and information for linking a thumbnail image selected/displayed on the list and the abstract information of all images is displayed.

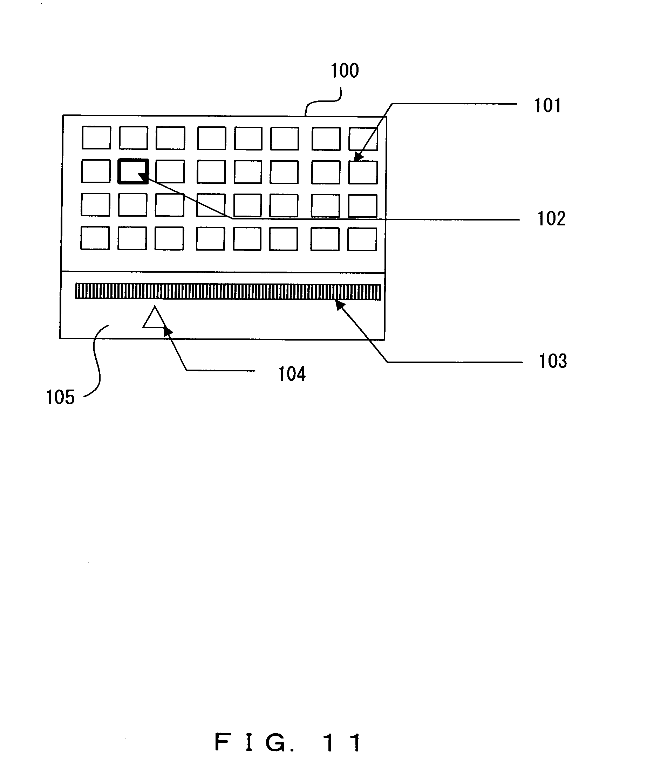

Fig. 11 shows a display screen that displays the list in the third embodiment (example 1) and the color bar/time bar at the same time so as to indicate the position of the image selected on the list. A list display screen 100 includes a list display area 101 for displaying a list including thumbnail images and an abstract information display area 105 for displaying abstract information covering all images, such as a color bar/time bar 103.

The position information on an image 102 being focused upon on the list can be displayed by using a mark such as a cursor 104 or a line or by changing a color on a position along the color bar/time bar 103.
Also, when a focused upon frame (hereinafter, a screen selected by a focused upon frame on a list is referred to as a focused image 102) is moved on a list, the position display such as the cursor 104 is also moved to the focused image. When the cursor 104 is moved along the color bar/time bar 103, the focused upon frame is also moved in an associated manner on the list so that a different image becomes the focused image 102. Upon this, if the cursor 104 is moved to an area where the list is not displayed, the list is scrolled so that an image corresponding to the movement destination of the cursor 104 is focused upon on the list.

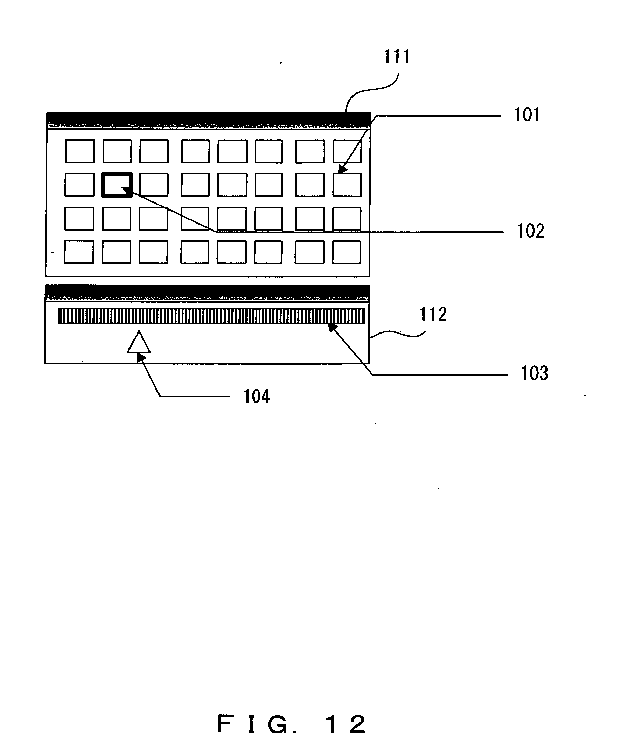

Fig. 12 shows a list display window 111 and an abstract information display window 112 displaying at the same time the list and the color bar/time bar on different screens so as to indicate the position of the image selected on the list, according to the third embodiment (example 2).

The present example is different from example 1 above in that the list display area based on thumbnail images and the abstract information display area for displaying abstract information covering all images such as the color bar/time bar 103 are divided into separate windows, i.e., into the list display window 111 and the abstract information display window 112.

The operations on the screen are the same as those in example 1, and the movement of the focused upon frame on the list window and the position of the cursor 104 on the abstract information display window 112 are associated with each other. Accordingly, when one of them is operated, the displayed content of the other is changed in an associated manner. In addition, the number of screens is not limited, and there can be as many screens as there are data items that are to be displayed.

### <Fourth Embodiment>

In the present embodiment, explanations will be given for displaying of what portion in abstract information covering all images the section of the listed images correspond to. There are various ways for displaying the portion, such as drawing a line or using a different color.

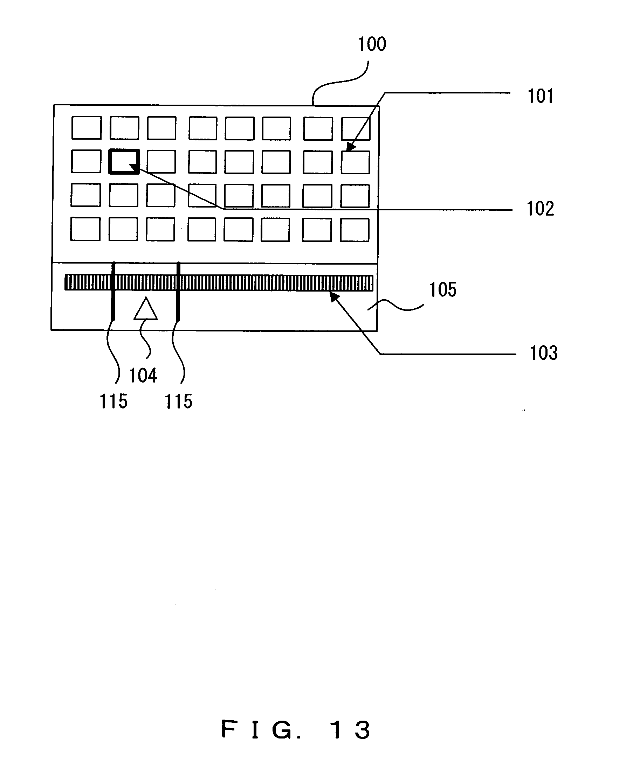

Fig. 13 shows a list display screen for displaying the section of a listed image on the color bar/time bar according to the fourth embodiment (example 1). As shown in Fig. 13, by drawing lines 115 on the color bar/time bar 103, the section of the listed image in the list display area 101 can be clearly expressed.

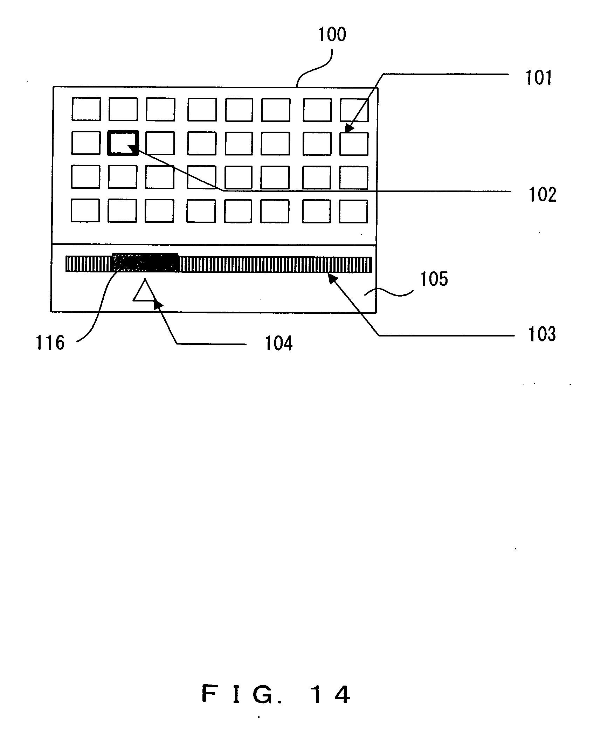

Fig. 14 shows a list display screen for displaying a section of a listed image on a color bar/time bar according to the fourth embodiment (example 2). As shown in Fig. 14, a section portion 116 corresponding to a listed image is displayed on the color bar/time bar 103 in a different color. Thereby, the section corresponding to a listed image can be clearly expressed.

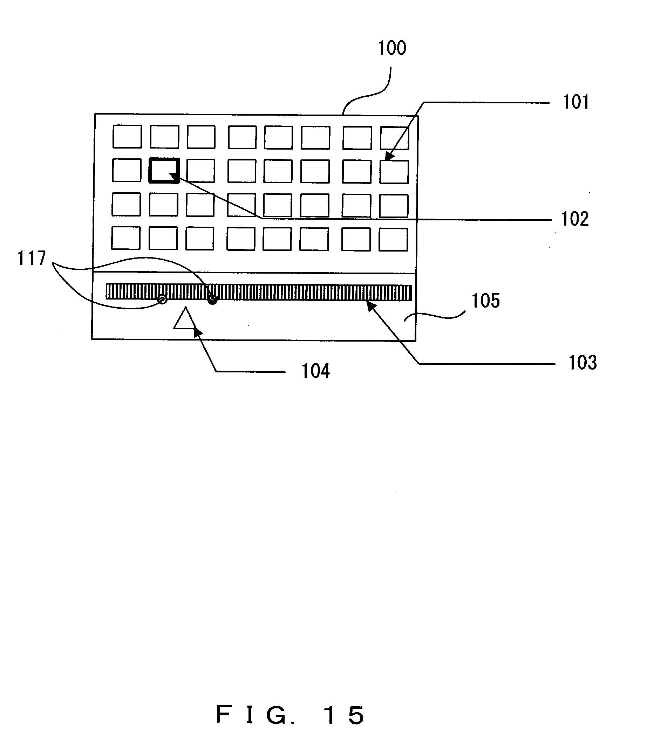

Fig. 15 shows a list display screen for displaying a section of a listed image on a color bar/time bar according to the fourth embodiment (example 3). As shown in Fig. 15, a section portion 116 corresponding to a listed image is displayed on the color bar/time bar 103 by marks 117. Thereby, the section corresponding to a listed image can be clearly expressed.

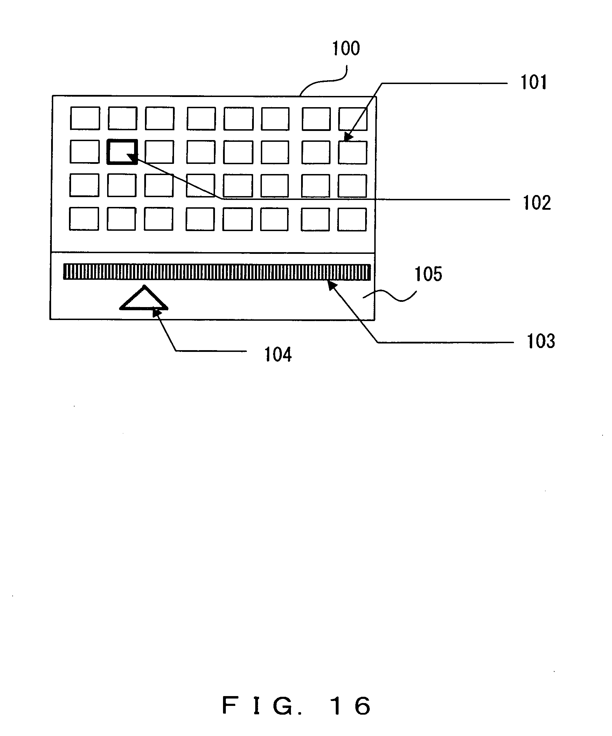

Fig. 16 shows a list display screen for displaying a section of a listed image on a color bar/time bar according to the fourth embodiment (example 4). In this example, the shape of the cursor 104 for indicating the position is changed so as to indicate the section of a listed image. In Fig. 16, an image of the section corresponding to the width of the cursor 104 is displayed. Thereby, the section corresponding to a listed image can be clearly expressed.

### <Fifth Embodiment>

In the present embodiment, explanations will be given for a configuration in which the list display screen can be scrolled, and the scrolling can be conducted not only in units of lines, but also in selected units, i.e., in units of plural lines, in units of screens, or in units of images.

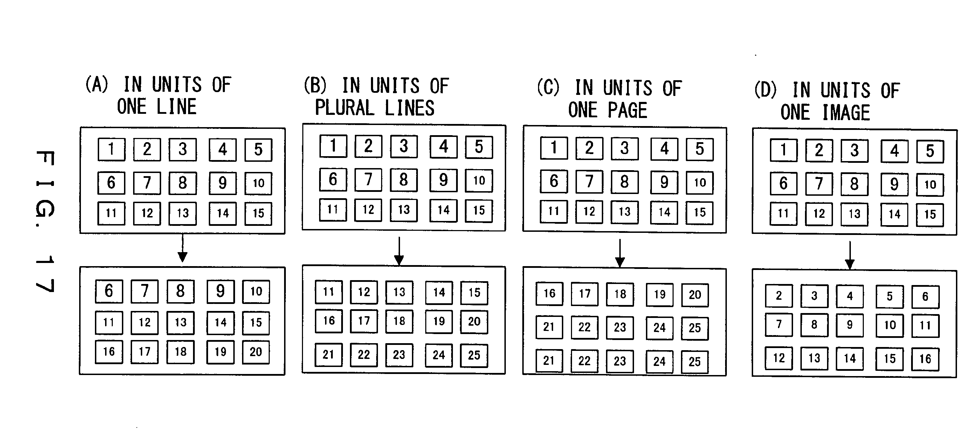

Fig. 17 shows a list display screen that can be scrolled in arbitrary units. Fig. 17(A) shows scrolling of the list in units of one line. Fig. 17(B) shows scrolling of the list in units of plural lines. Fig. 17(C) shows scrolling of the list in units of screens (pages). Fig. 17(D) shows scrolling of the list in units of an image.

As described above, units of scrolling can be set in advance. Accordingly, the amount of scrolling of images when scrolling operations are performed varies depending upon the set values.

### <Sixth Embodiment>

In the present embodiment, explanations will be give to (1) selecting/displaying distinguishing images from the first of successive images, (2) selecting/displaying characteristic images before and after the image being currently displayed on the moving-image display screen, (3) selecting/displaying distinguishing images in a range including the image being currently displayed on the moving-image display screen, performed when the screen for displaying moving images transitions to the list display screen.

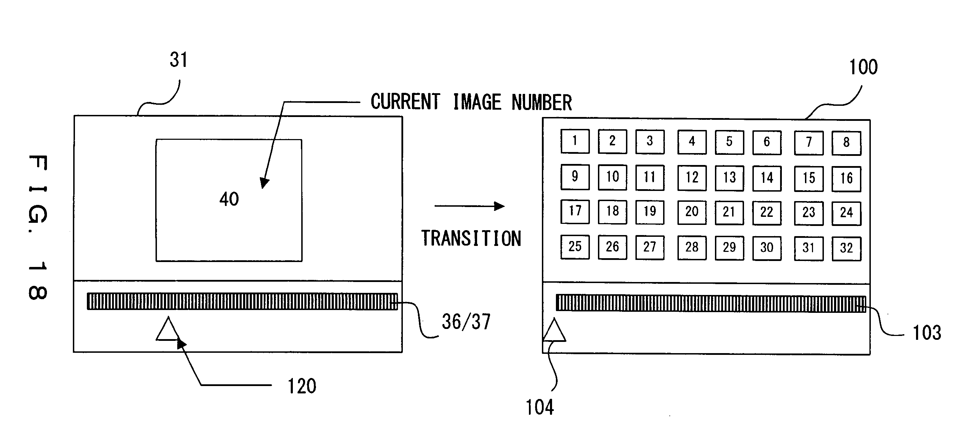

Fig. 18 shows a transition from the moving-image display screen to the list display screen according to the sixth embodiment (example 1). The moving-image display screen, on the left side, corresponds to the window 31 in Fig. 3. A cursor 120 indicates the current operation reproducing position on the color bar 36/time bar 37 in the window in Fig. 3.

In the present example, when the moving-image display screen 31 transitions to the list display screen 100, characteristic images are listed starting from the top of the successive images regardless of whether the current moving image is being reproduced.
Thereby, when the moving-image display screen 31 transitions to the list display screen 100, distinguishing images can be selected/displayed from the first of successive images under any situation. Also, images can be confirmed from the first of successive images regardless of operations being conducted.

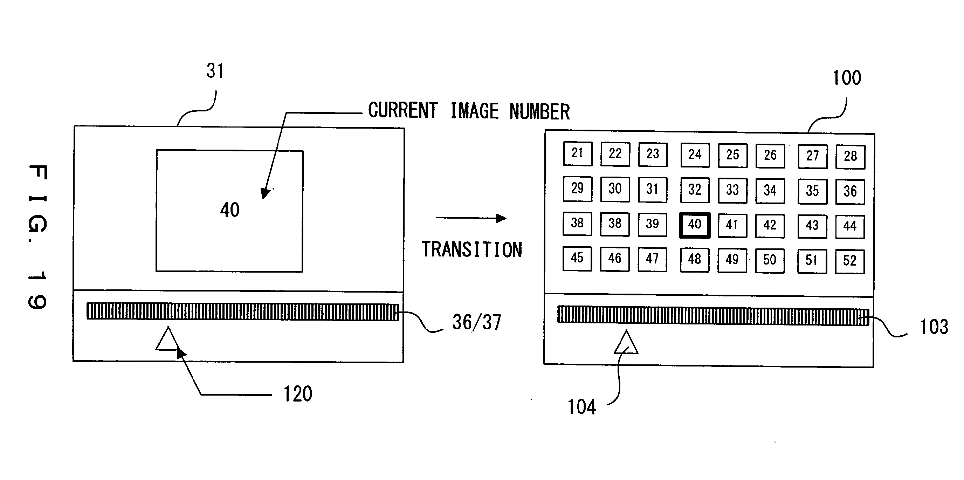

Fig. 19 shows a transition from the moving-image display screen to the list display screen according to the sixth embodiment (example 2). In the present example, when the moving-image display screen 31 transitions to the list display screen 100, a prescribed number of images before and after the position of the image being currently displayed in the moving-image display screen 31 are displayed. Thereby, characteristic images before and after the currently observed image can be observed in the moving-image display screen 31.

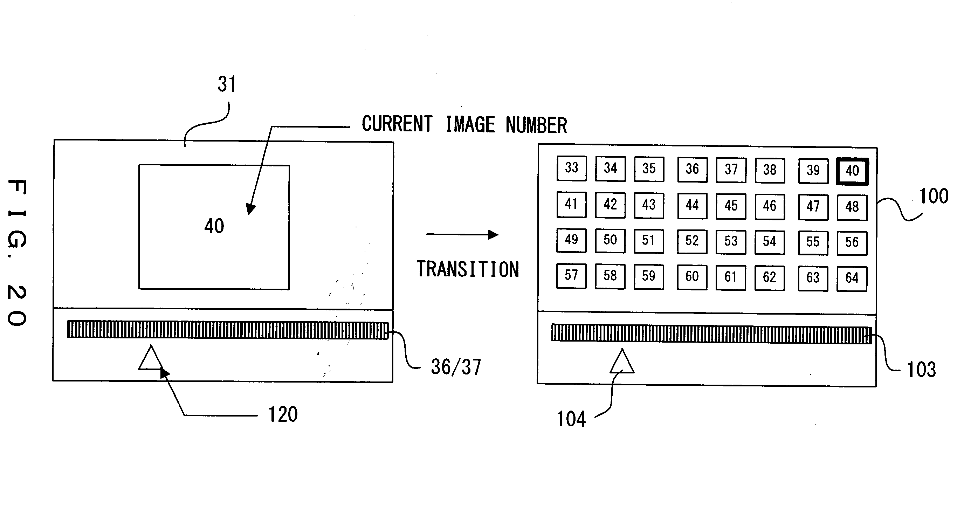

Fig. 20 shows a transition from the moving-image display screen 31 to the list display screen 100 according to the sixth embodiment (example 3). In the present example, when the moving-image display screen 31 transitions to the list display screen 100, images on the pages including the current image position are listed and displayed. Thereby, the same group of images can be selected/displayed when the cursor is in a prescribed section regardless of the kinds of operations being currently conducted.

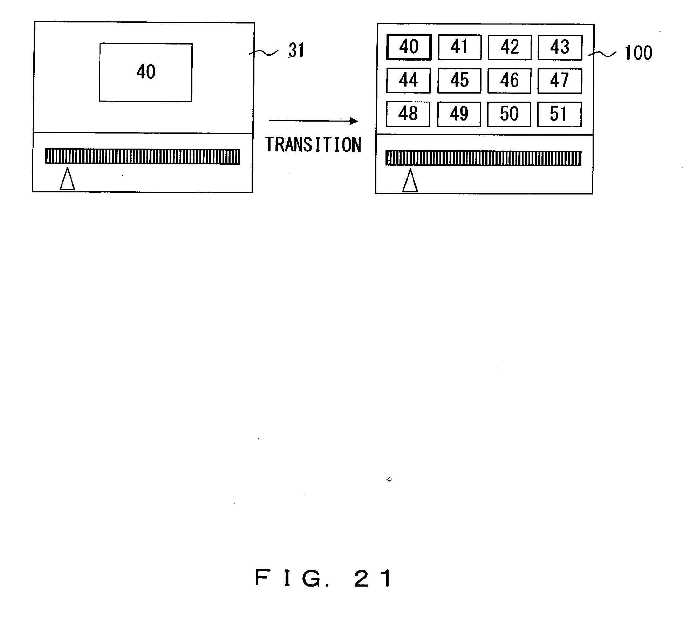

Fig. 21 shows a transition from the moving-image display screen 31 to the list display screen 100 according to the sixth embodiment (example 4). In the present example, when the moving-image display screen 31 transitions to the list display screen 100, images that are being displayed are listed and displayed from the top of the page.

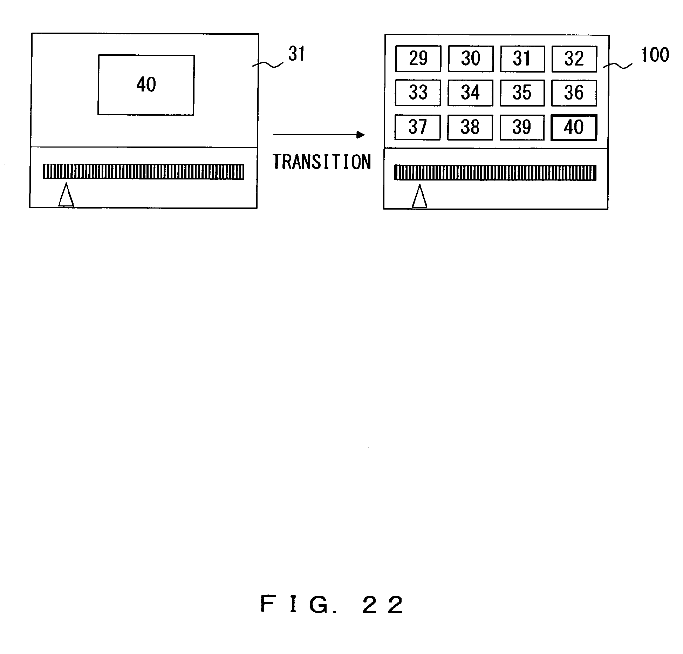

Fig. 22 shows a transition from the moving-image display screen 31 to the list display screen 100 according to the sixth embodiment (example 5). In the present example, when the moving-image display screen 31 transitions to the list display screen 100, images that are being displayed are listed and displayed from the last page.

Also, it is possible to set which one is to be selected from among the sixth embodiment (example 1) shown in Fig. 18, the sixth embodiment (example 2) shown in Fig. 19, the sixth embodiment (example 3) shown in Fig. 20, the sixth embodiment (example 4) shown in Fig. 21, and the sixth embodiment (example 5) shown in Fig. 22.

### <Seventh Embodiment>

In the present embodiment, explanations will be given for (1) displaying the first of successive images, (2) displaying images before being displayed in a list, and (3) displaying images selected on the list display screen, performed when the list display screen 100 transitions to the moving-image display screen 31.

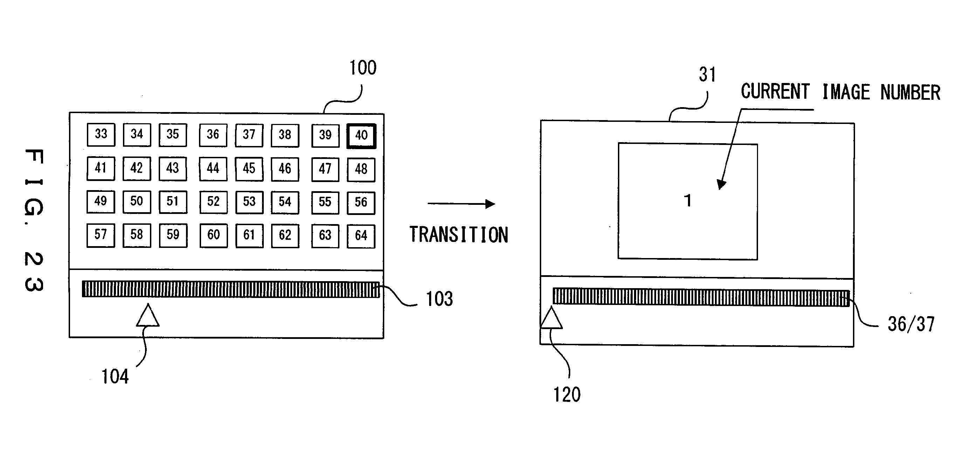

Fig. 23 shows a transition from the list display screen to the moving-image display screen according to the seventh embodiment (example 1). In Fig. 23, when the list display screen 100 transitions to the moving-image display screen 31, the first of successive images is selected/displayed. Thereby, images can be confirmed from the first of successive images regardless of the kinds of operations being currently conducted.

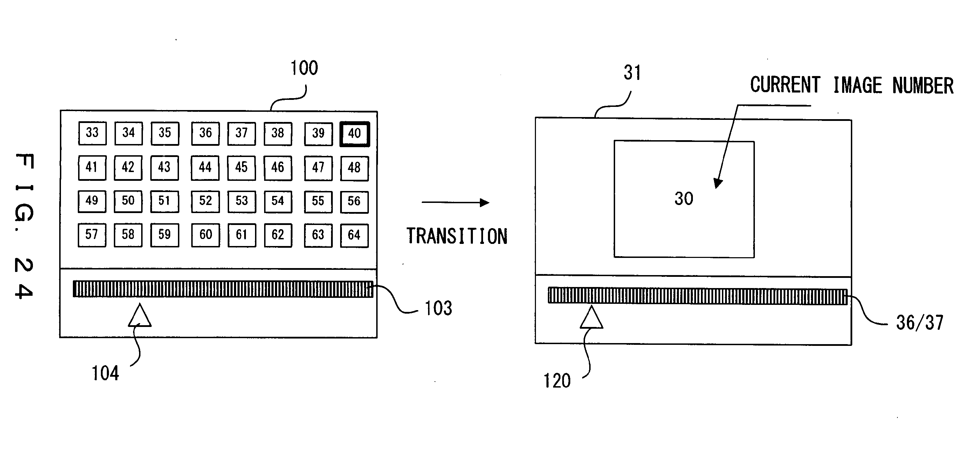

Fig. 24 shows a transition from the list display screen to the moving-image display screen according to the seventh embodiment (example 2). In Fig. 24, when the list display screen 100 transitions to the moving-image display screen 31, images before the screen transitions to the list display screen are selected/displayed. Thereby, users can return to operations that had been conducted before the list display screen is displayed regardless of the kinds of operation being currently conducted.

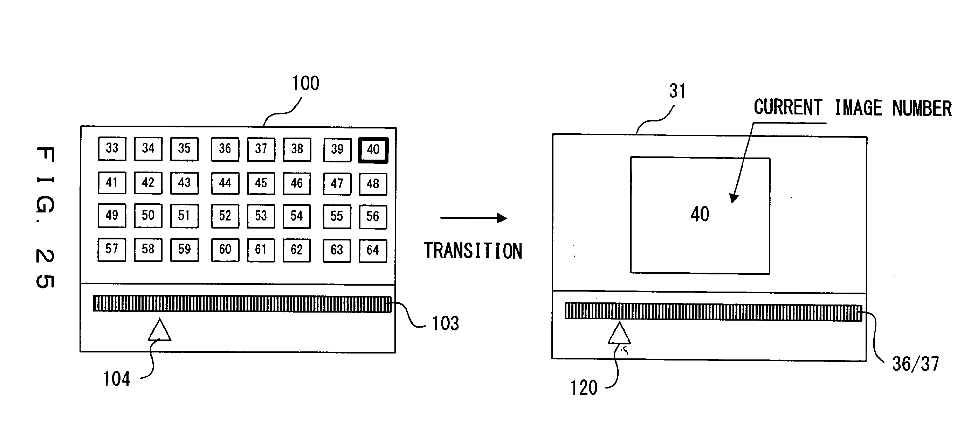

Fig. 25 shows a transition from the list display screen to the moving-image display screen according to the seventh embodiment (example 3). In Fig. 25, when the list display screen 100 transitions to the moving-image display screen 31, the image focused upon currently on the list is selected/displayed. Thereby, images can be confirmed starting from the image focused upon on the list display screen.

### <Eighth Embodiment>

In the present embodiment, explanations will be given for a configuration in which when images selected/displayed on the list display screen are to be displayed over plural pages, display lines for indicating the borders between pages are displayed on abstract information (color bar/time bar) for all images. Various ways can be used for displaying the borders, such as drawing lines or using a different color.

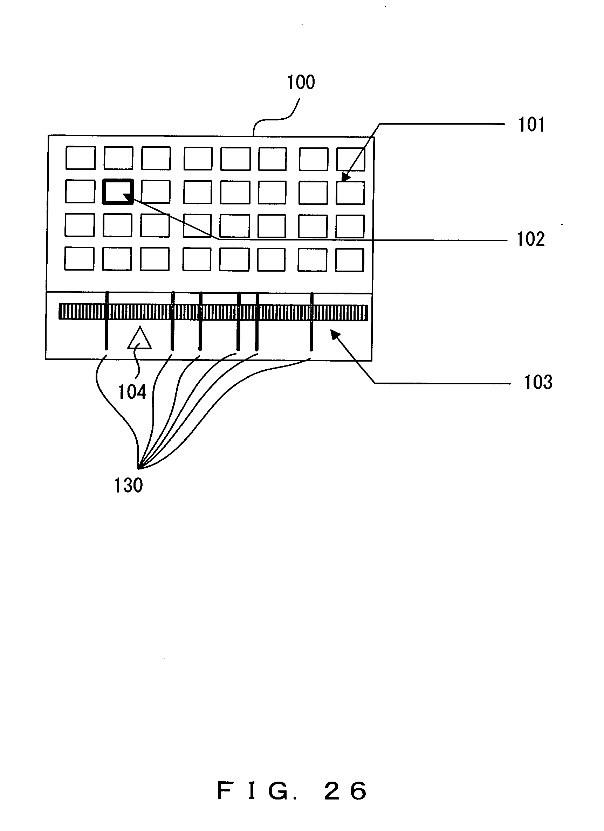

Fig. 26 shows a list display screen on which page sections for a listing display are displayed on the color bar/time bar according to the eighth embodiment. As shown in Fig. 26, portions between lines 130 on the color bar/time bar 103 correspond to the displayed sections of the listed images.

Thereby, page sections on a list can clearly be displayed. Additionally, borders between pages can be displayed not only in the form of lines but also, for example, in the form of marks, as shown in the fourth embodiment.

### <Ninth Embodiment>

In the present embodiment, explanations will be given for a list display screen on which a check mark can be placed on an image that attracts a user's attention on the list display screen.

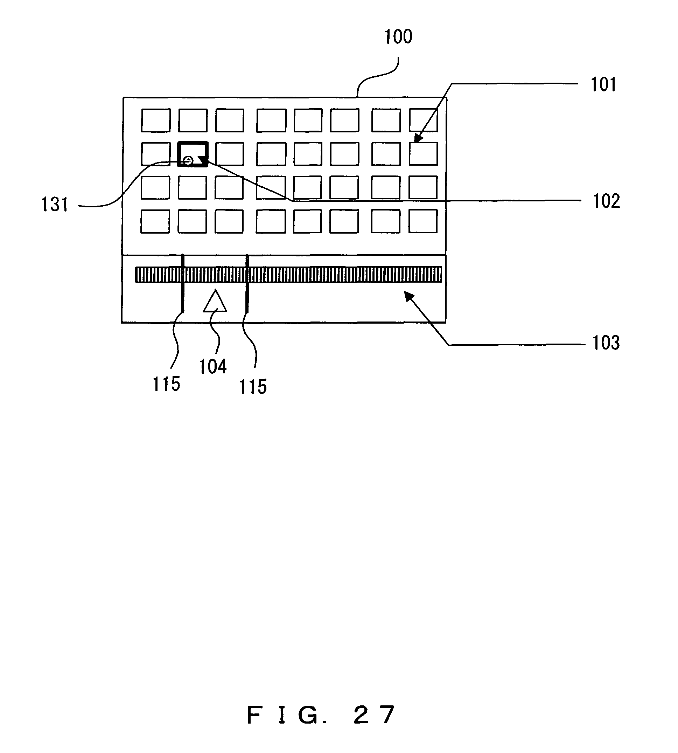

Fig. 27 shows a list display screen on which a check mark can be placed on an interesting image on the list display screen according to the ninth embodiment (example 1). In the present example, it is possible to select an interesting image on the list display screen 100 so as to place a check mark 131 on the interesting image.

As shown in Fig. 27, an image on which a check mark is placed can be discriminated by a display added to a part near the image, such as a mark or a frame in a different color. A mark for discriminating a checked image can be any mark regardless of color or shape. Such a mark can be in the form of text.

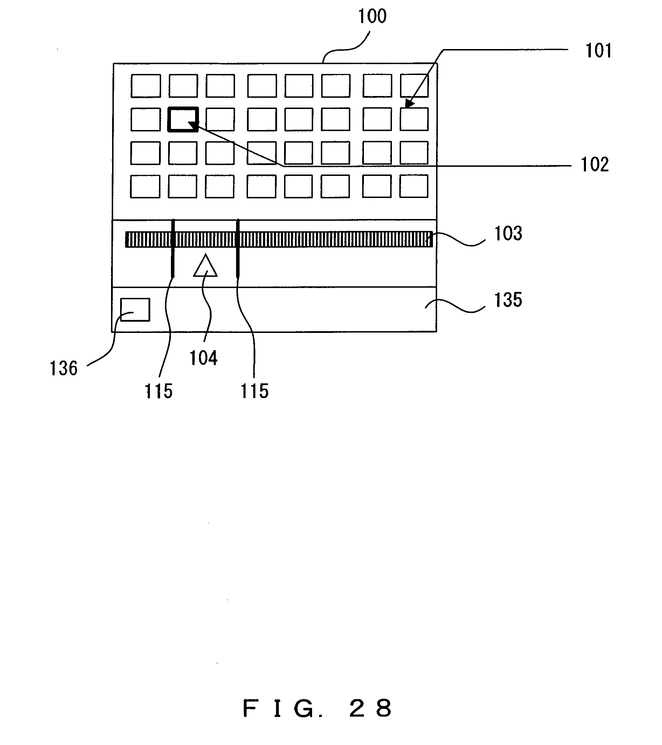

Fig. 28 shows a list display screen on which a check mark can be placed on an interesting image on the list display screen according to the ninth embodiment (example 2). In the present example, similarly to Fig. 27, it is possible to select an interesting image on the list display screen 100 so as to place a check mark on the interesting image.

As shown in Fig. 28, the image on which a check mark is placed in the list display area 101 is registered in a sub-display area 135 as a thumbnail image 136. Additionally, a mark for association with a listing display can be any mark, regardless of color or shape. Such a mark can be in the form of text.

When a check mark is placed on an arbitrary image being listed and displayed, discrimination information is added to a part near a thumbnail image for a listing display, such as a mark or a differently-colored frame (for emphasis), and also a sub-display area can be used for displaying the checked image.

### <Tenth Embodiment>

In the present embodiment, explanations will be given for a configuration in which an interesting image that is checked on the list display screen so as to be displayed in the sub-display area is scrolled in connection with the list display area being scrolled. For this, it is possible to select whether or not to display an image when that image is not in the image section displayed as a list.

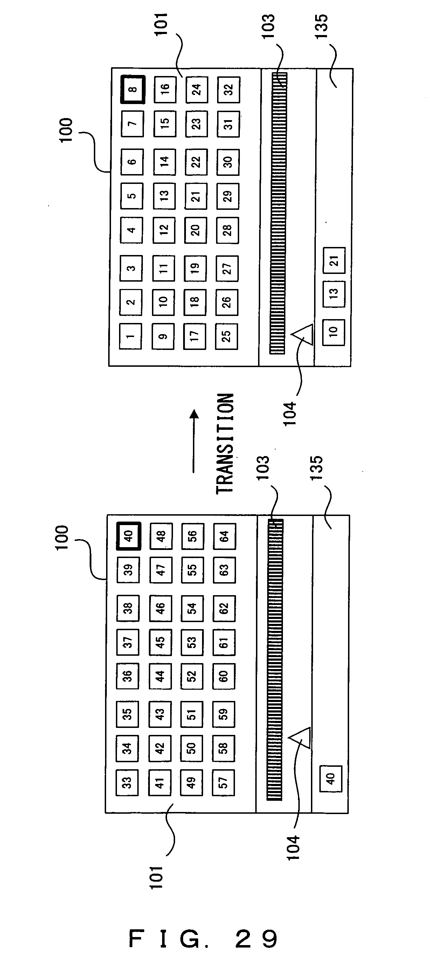

Fig. 29 shows changing of a display state of an interesting image in a sub-display area for displaying a list according to the tenth embodiment (example 1). As shown in Fig. 29, an interesting image checked in the list display area 101 is displayed as a thumbnail image in the sub-display area 135.

Only an interesting image in the section of the image being currently displayed in the list display area 101 is displayed in the sub-display area 135. Accordingly, when the list display area 101 is scrolled so that all the images are replaced, the thumbnail image in the sub-display area 135 is also replaced in response to that replacement. Also, it is possible to set whether or not to replace the sub-display area 135 in response to a replacement of images in the list display area 101.

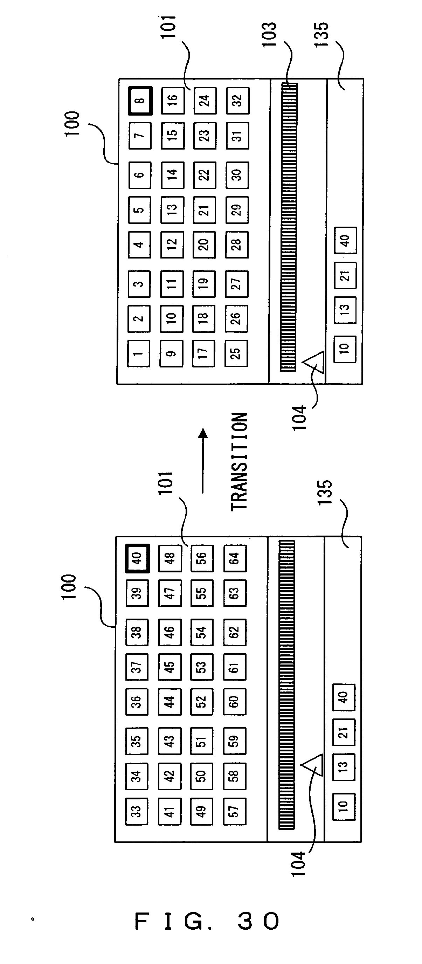

Fig. 30 shows a changing of a display state of an interesting image in a sub-display area for displaying a list according to the tenth embodiment (example 2). As shown in Fig. 30, an interesting image checked in the list display area 101 is displayed as a thumbnail image in the sub-display area 135.

In the present example, all interesting images in a section not being displayed in the list display area 101 are also displayed in the sub-display area 135. Accordingly, even when the list display area 101 is scrolled so that all images are replaced, there are no changes in thumbnail images in the sub-display area.

However, when there are a number of thumbnail images that cannot be included in the sub-display area 135, the thumbnail image closest to the image display position is displayed at the center. In addition, it is possible to set whether or not to replace an image in the sub-display area 135 in response to a replacement of images in the list display area 101.

### <Eleventh Embodiment>

In the present embodiment, it is possible to perform setting so that when there is an image that has already been selected as a characteristic image used for moving-image display, that image can be displayed in a list even when the image is not selected by the algorithm selected for displaying the list. Also, explanations will be given for placing marks on those images by means of marks or differently colored frames for displaying that the image is selected by a different algorithm.

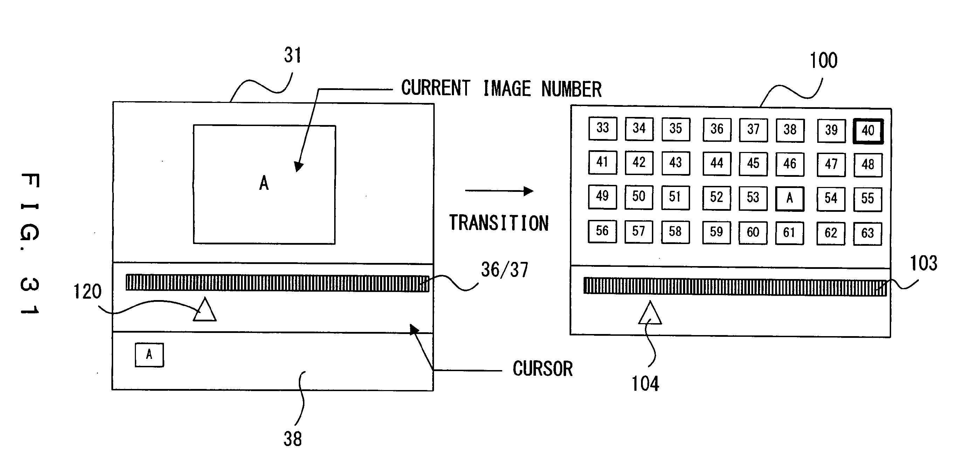

Fig. 31 shows the addition of an image selected on the moving-image display screen to the list display screen according to the eleventh embodiment. As shown in Fig. 31, it is possible to perform setting so that even when an image selected in the moving-image display screen 31 is an image not to be selected by the algorithm selected for displaying a list (lesion detection or the like), the image is displayed as a thumbnail image on the list display screen. This selected image may be distinguished by a mark indicating that the image is not an image selected by the algorithm selected for displaying a list.

### <Twelfth Embodiment>

In the present embodiment, explanations will be given for a configuration in which the position of each image to be displayed on the list display screen is overlapped and displayed on the abstract information covering all images. Thereby, portions from the entire image that correspond to the images extracted by the selected algorithm can be determined.

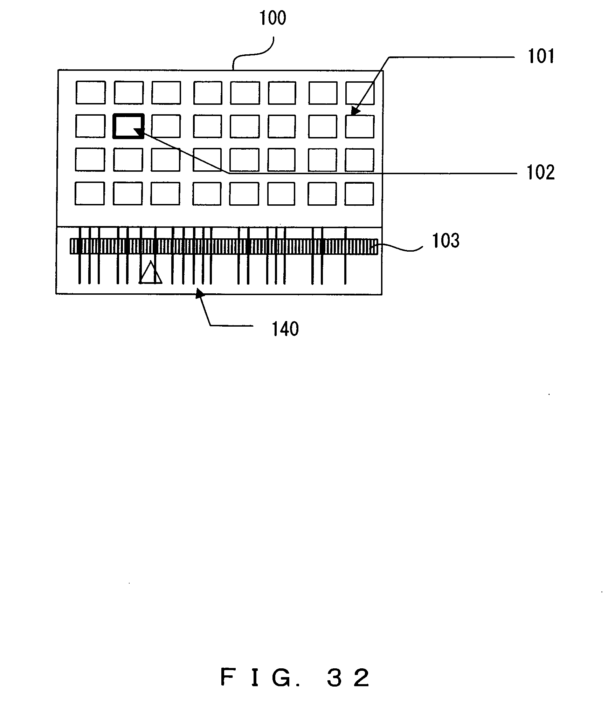

Fig. 32 shows a list display screen on which marks of images extracted for a list display are placed on the color/time bar according to the twelfth embodiment. As shown in Fig. 32, marks are placed in the form of lines 140 at the positions on the color bar/time bar 103 that correspond to the images extracted for a listing display. Thereby, positions of images extracted for a listing display can be clearly displayed on the color bar/time bar 103. The positions of images can be displayed not only by lines but also by the marking methods explained in the fourth embodiments.

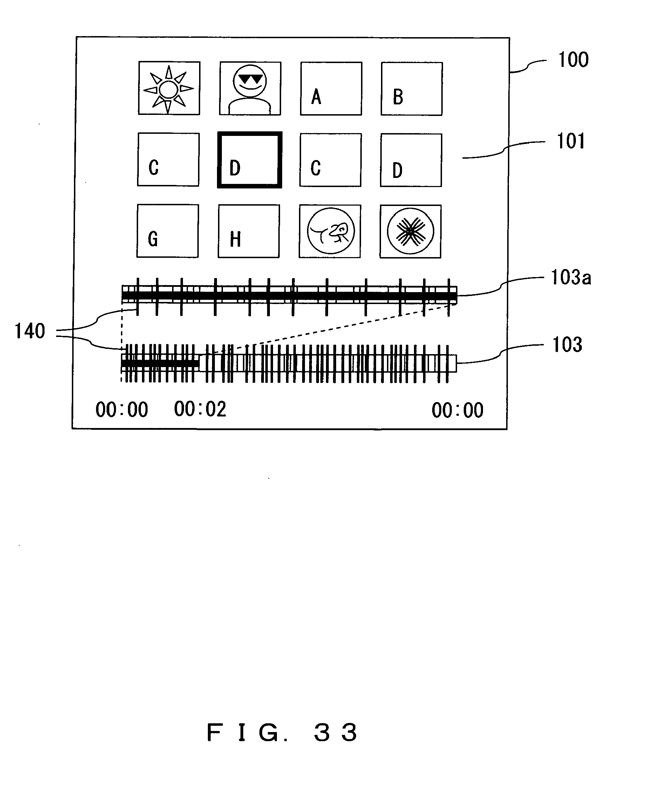

Fig. 33 shows an example of a list display screen for displaying a desired range on the color bar/time bar having marks at positions corresponding to images extracted for a listing display according to the twelfth embodiment (variation example 1). In Fig. 32, marks are placed at positions corresponding to respective images extracted for a listing display on the color bar/time bar displaying all time-series images. In the present variation example, a desired range of the color bar/time bar 103 displaying all time-series images shown in Fig. 32 is selected, and the portion of the color bar/time bar corresponding to the selected range is displayed in an enlarged manner (a color bar/time bar 103a). The list display area 101 displays only images corresponding to the marks on the color bar/time bar 103a.

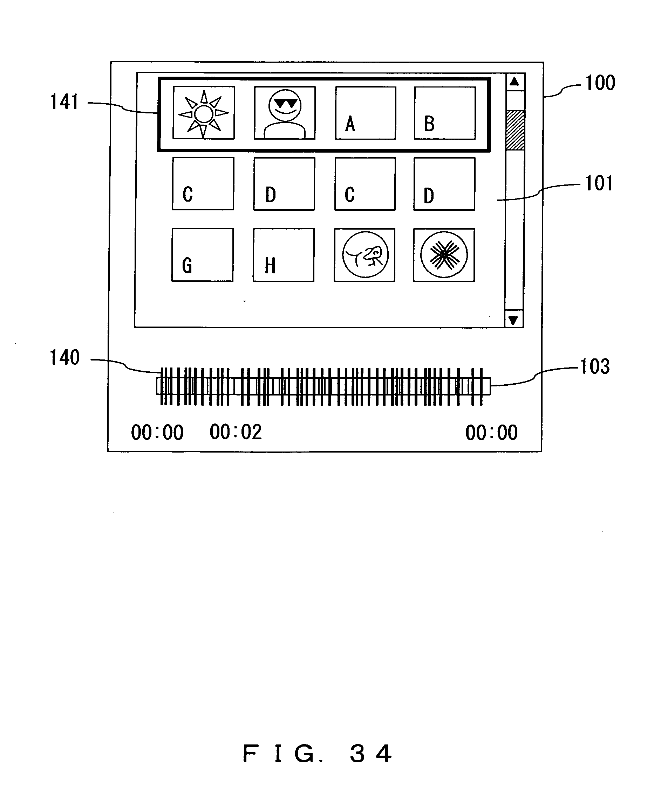

Fig. 34 shows a list display screen according to the twelfth embodiment (variation example 2). It is possible to specify a range in units of lines as denoted by numeral 141 in Fig. 34 or to specify a range in units of pages on the list display screen (Fig. 32) in which a desired range is not narrowed to a desired range.

### <Thirteenth Embodiment>

In the present embodiment, an example will be shown in which a digest image detected by a prescribed detection algorithm as explained in the above embodiment and time-series images before and after that digest image (images for a case when the detection algorithm is not executed) are displayed in the list display area 101.

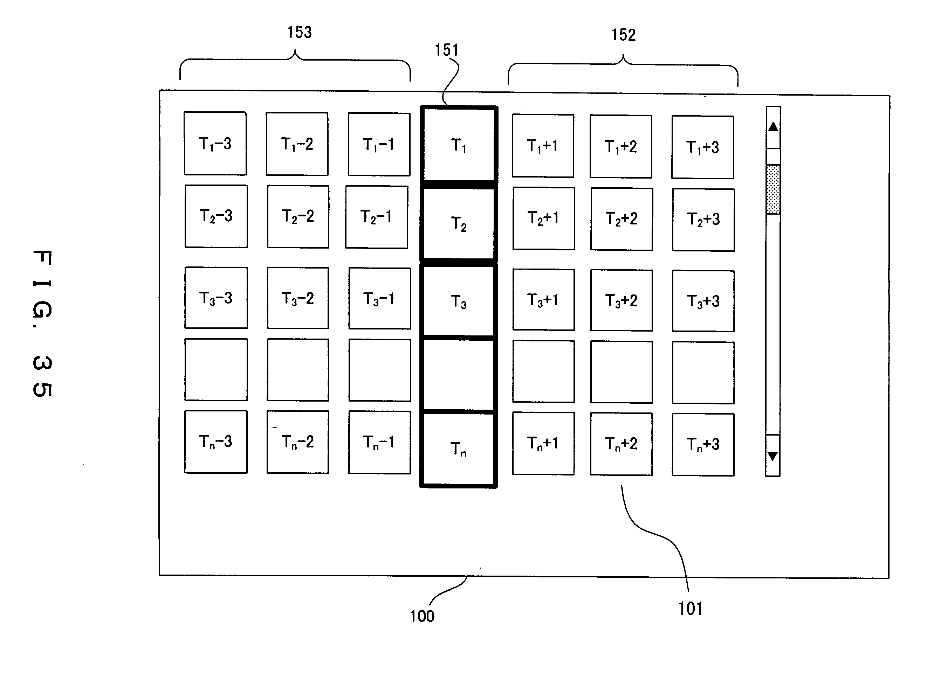

Fig. 35 shows the list display screen 100 according to the present embodiment. In the list display area 101, digest images T₁, T₂, T₃,..... Tₙ are displayed from the top in time series in a center column 151. On both sides of the respective digest images T₁, T₂, T₃, ..... Tₙ, time-series images before and after each of the digest images T₁, T₂, T₃,.....Tₙ are displayed. Specifically, an image area 153 displays, in time series, at least one image that was captured in time before the digest image. An image area 152 displays, in time series, at least zero images that were captured in time after the digest image. The image area 151 may display the digest images in an enlarged manner or may add marks to the digest images so that the images being displayed are digest images.

According to the present embodiment, it is possible to confirm time-series images before and after the digest images detected by a prescribed detection algorithm. Thereby, images before and after characteristic images can be displayed in a listed manner, leading to an increase in observation efficiency.

### <Fourteenth Embodiment>

In the present embodiment, explanations will be given for an enlarged display of images performed when a mouse cursor 161 is on one of the respective images in the list display area 101.

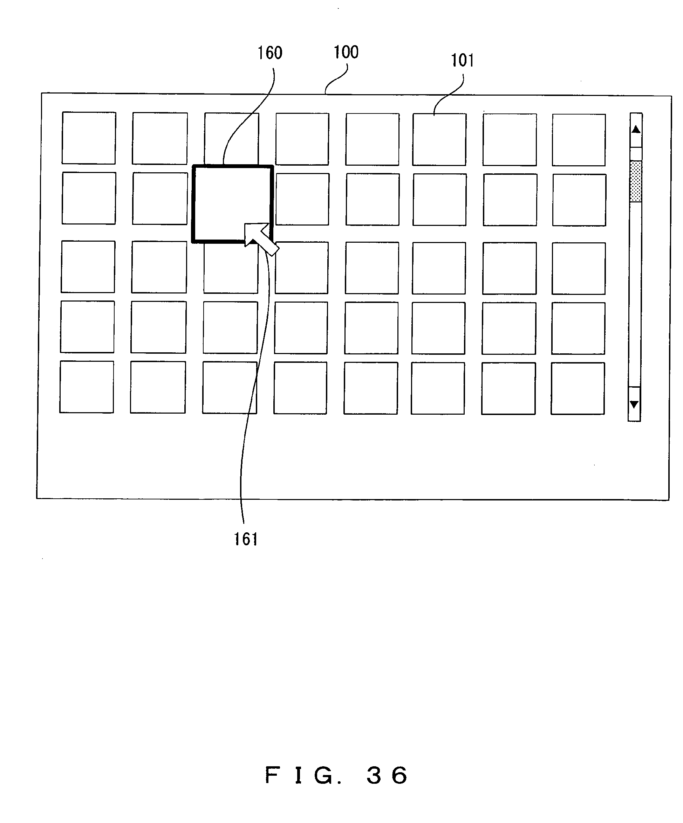

Fig. 36 shows an enlarged display of an image performed when the mouse cursor 161 is moved to an image displayed in the list display area 101. The image closest to the mouse cursor 161 is displayed in an enlarged manner as denoted by numeral 160. When the enlarged display denoted by numeral 160 is being displayed, attribute information may be displayed together with it.

According to the present embodiment, when a cursor is on an interesting image among images displayed in a listed manner, that image can be displayed in an enlarged manner. Thus, that interesting image can be displayed in an enlarged manner without opening another window, thereby increasing observation efficiency.

### <Fifteenth Embodiment>

In the present embodiment, explanations will be given for setting of a threshold value for detecting a digest image.

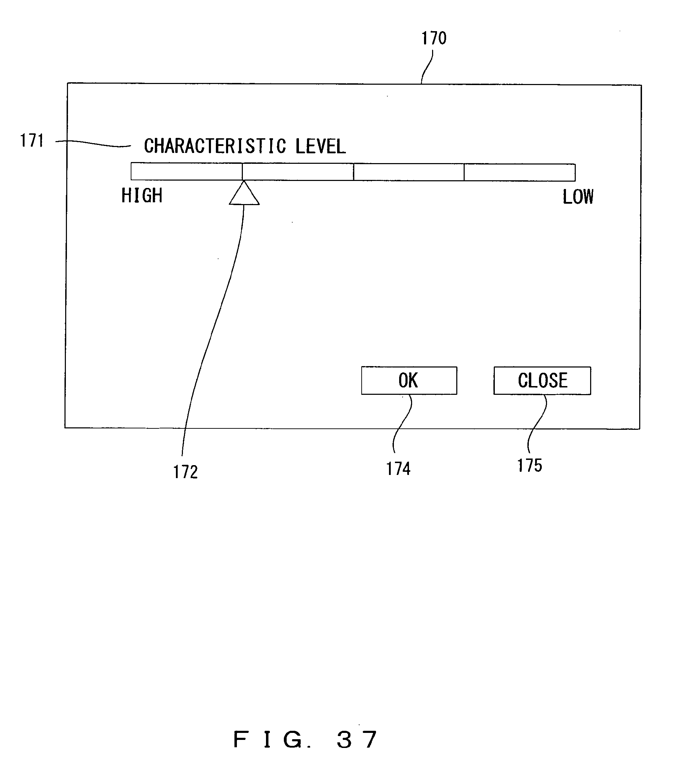

Fig. 37 shows a detection condition setting screen for setting a condition for detecting a digest image according to the present embodiment. As explained in the first embodiment, when switch 2 (41b) is pressed, a digest image is detected from time-series images on the basis of similarity, and a detection condition setting window 170 can set this similarity.

In Fig. 37, "Characteristic Level" 171 corresponds to the above similarity. As the "Characteristic Level" 171, one of five characteristic levels can be selected by using, for example, a cursor 172. As described above, whether or not two images are similar to each other is determined on the basis of whether or not a difference in pixel value between the two images is higher than a threshold value. Accordingly, the higher the set Characteristic level is, the greater the difference is.

When setting content of the above detection condition is to be set and updated, an "OK" button 174 is pressed. When the screen is to be closed, a "Close" button 175 is pressed.
According to the present embodiment, a detection condition can be set by a user when detection is to be performed, and accordingly detection that is more suitable for the user can be performed.

### <Sixteenth Embodiment>

In the present embodiment, explanations will be given for a configuration in which a group of images in a particular section is specified in a group of time-series images, and time-series images in the specified section are displayed in a listed manner.

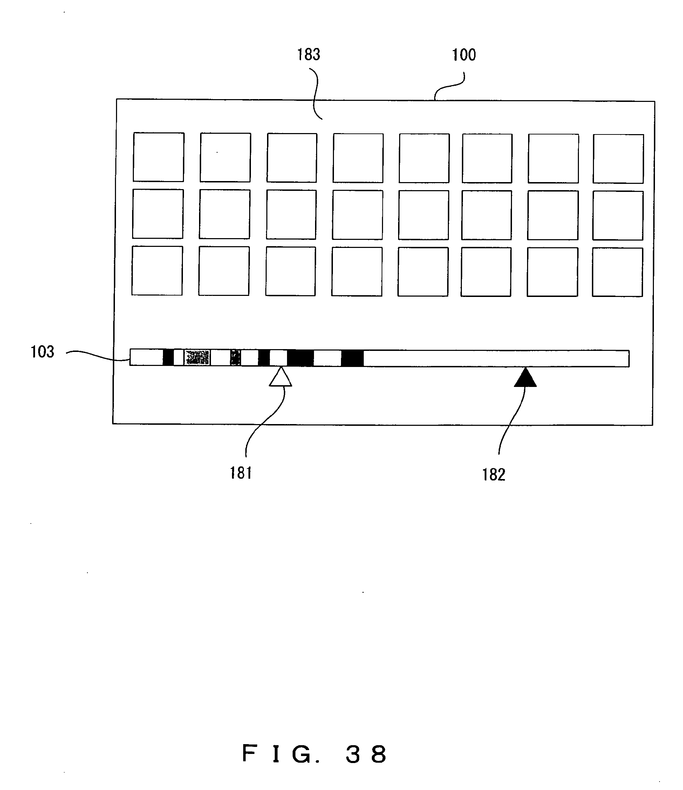

Fig. 38 shows the list display screen 100 in the present embodiment in which an image group in a particular section is specified in a time-series image group and time-series images in the specified range are displayed.
On the color bar/time bar 103, the starting point and the ending point of a section is specified by a particular-section-starting cursor 181 and a particular-section-ending cursor 182, respectively. Then, time-series images in the specified section are detected, and the detected images are displayed in a listed manner as denoted by numeral 183. Upon this occurring, time-series images out of the specified section may be included in the displayed list.

According to the present embodiment, it is possible to display time-series images in a particular section. Thus, by setting a section in which images of an arbitrary organ were captured, it is possible to display only time-series images of that organ.

Thus, it is also possible to set a threshold value of characteristic detection in a particular section that has been set when the sixteenth embodiment is combined with the fifteenth embodiment. Thereby, a list of digest images in that particular section can be displayed.

### <Seventeenth Embodiment>

In the present embodiment, explanations will be given for a configuration in which plural particular sections of time-series images are specified and a threshold value is set for detecting digest images for each of the sections specified.

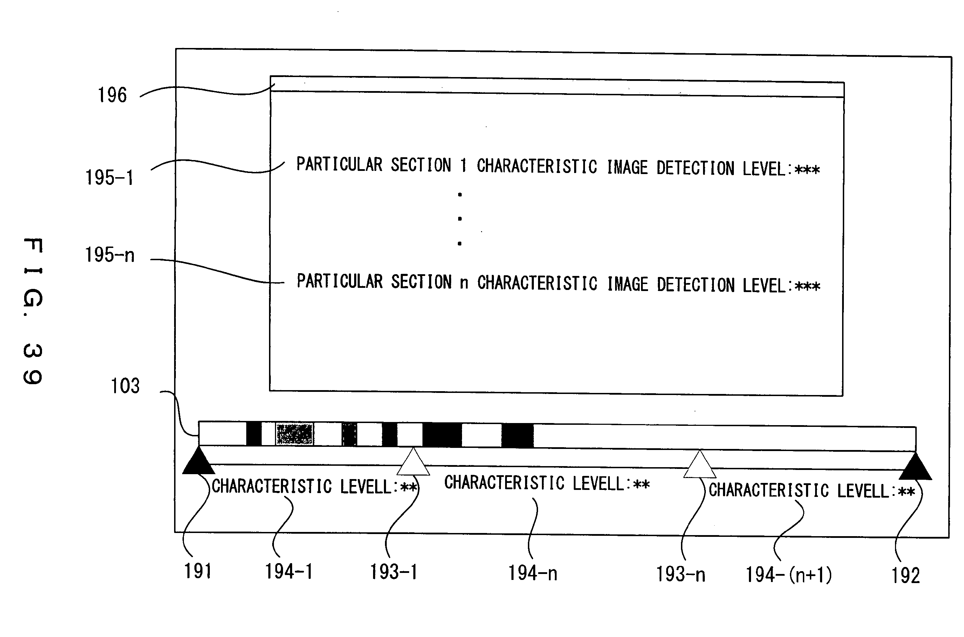

Fig. 39 shows a detection condition setting screen for detecting a digest image for each organ (site) or each particular section. The window in Fig. 39 has a particular-section-starting cursor 191 for specifying the starting point of a section, a particular-section-ending cursor 192 for specifying the ending point of the section, specifying cursors 193-1,..... 193-n for specifying respective sections, a setting screen 196 for setting a characteristic level (threshold value) for detecting characteristic images in specified sections, and setting fields 195-1,..... 195-n for setting threshold values for the respective sections.

First, respective sections are specified by the particular-section-starting cursor 191, the particular-section-ending cursor 192, and the specifying cursors 193-1,..... 193-n. Next, the characteristic levels (threshold values) for the corresponding sections specified are set in the setting fields 195-1,..... 195-n in the setting screen 196.

When a digest image is detected on the basis of the above setting content, digest images in each section are detected, and are displayed on the list display screen. Upon this occurring, time-series images out of the specified sections may be included in the displayed list.

According to the present embodiment, a detection threshold value can be set for each organ (site) or for each particular section. This makes it possible to respond to a problem in which a different threshold value for detecting characteristics has to be set for each site due to the fact that a capsule endoscope moves at different speeds depending upon the organs (sites) it is passing through.

### <Eighteenth Embodiment>

In the present embodiment, explanations will be given for a configuration in which pages in a listing display are turned sequentially after a time period set by a user has elapsed.

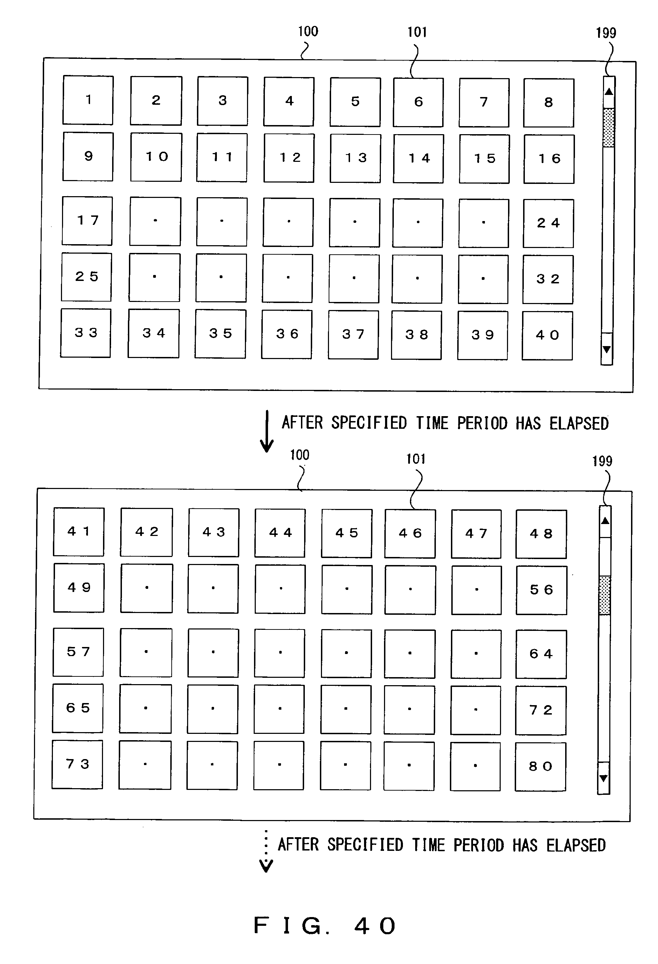

Fig. 40 shows a sequential turning of pages in a listing display performed after a time period set by a user has elapsed. This is performed when a listing display covers plural pages. Numeral 199 denotes a scroll bar. When pages are tuned, the scroll bar is also moved.

According to the present embodiment, when a list to be displayed cannot be included in one page, pages are turned automatically, and thereby it is possible to relieve users of the burden of scrolling the screen.

### <Nineteenth Embodiment>

In the present embodiment, explanations will be given for specifying of an observation time period as a detection condition in order to determine a threshold value for detecting digest images.

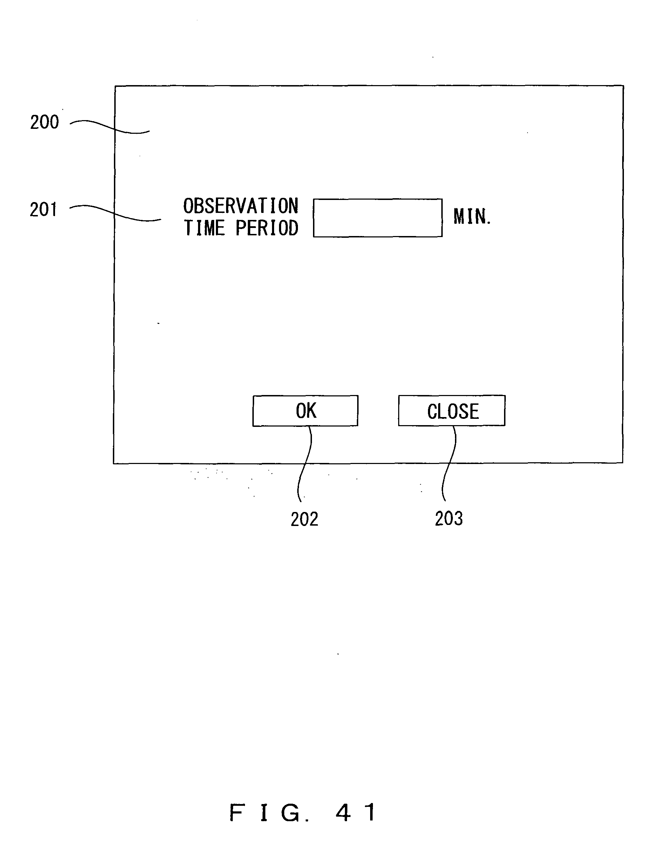

Fig. 41 shows a detection condition setting screen 200 for setting an observation time period according to the present embodiment. The detection condition setting screen 200 includes an "Observation Time Period" input box 201, an "OK" button 174, and a "Close" button 175. When the "Close" button 175 is pressed, the detection condition setting screen 200 is closed.

When a time period (in units of minutes) is set in the "Observation Time Period" input box 201 and the "OK" button 174 is pressed, digest images can be reproduced within the set observation time period. Specifically, the number of images to be observed is calculated on the basis of the set time period information and the frame rate so that the manipulator can perform observation within the set time period. A threshold value of similarity (characteristic level) used for detection from time-series images is determined so that the calculated number of digest images can be obtained.

According to the present embodiment, busy observers can observe images that are at a high characteristic level over a finite period of time.

### <Twentieth Embodiment>

In the present embodiment, explanations will be given for a configuration in which the main display area 32 described in Fig. 3 displays a digest image as the main display image 33, and time-series images before and after the digest image are displayed on the right and left (or higher and lower) sides of the main display image 33.

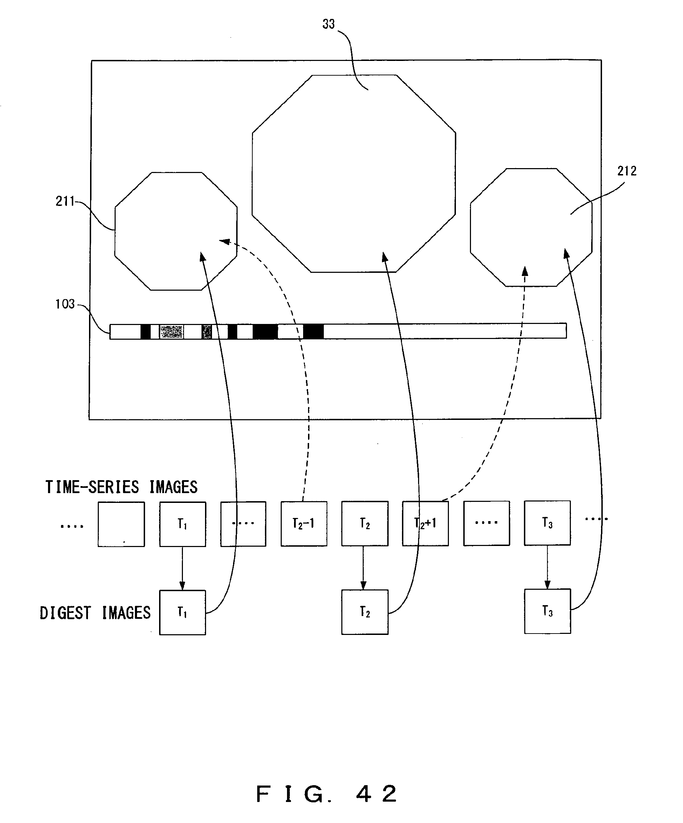

Fig. 42 shows an observation screen for an endoscope system according to the present embodiment. In the window 31, the main display area 32 for displaying a main display image or the like and the color bar/time bar 103 are displayed.

In the main display area 32, the main display image 33 is displayed at the center, and sub-display images 211 and 212 are displayed on the right and left sides of the main display image 33. While, for example, digest image T₂ is reproduced as the main display image 33, digest images T₁ and T₃, which are images before and after digest image T₂, are reproduced as the sub-display images 211 and 212.

When a digest image has ceased to be reproduced, digest image T₂ is displayed as the main display image 33 as the display stop instruction. At that time, time-series images T₂-1 and T₂+1, which are images before and after digest image T₂, are displayed as the sub-display images 211 and 212, respectively.

According to the present embodiment, time-series images before and after that digest image can be displayed at the same time that a digest image is displayed, increasing observation efficiency.
According to embodiments of the present invention, when case data is confirmed on a displayed list, the data can be checked together with the entire positional relationship so that time-series information about images can be easily understood. Also, by achieving closer association between existing functions, list displaying, and moving-image displaying, characteristic sites can be detected from among a series of images at a higher speed.

Additionally, in all the embodiments of the present invention described herein, various modifications are allowed without departing from the spirit of the invention. Also, the two or more embodiments described above can be combined in an arbitrary manner as long as the combined embodiments are feasible.

## Claims

1. An image processing apparatus, comprising:
obtainment means for obtaining time-series image data, which is image data captured in time series by a capsule endoscope;
image detection means for detecting prescribed image data from the obtained time-series image data; and
display control means for displaying a list of detection image data, which is the detected image data.

2. The image processing apparatus according to claim 1, wherein:
the image processing apparatus further comprises:
display instruction input means to which an instruction to the display control means is input; and
the display instruction input means comprises:
detection method selection means for selecting a detection method used when detection is performed on the basis of the image detection means;
list display selection means for displaying a list of the image data by using the display control means; and
moving image reproduction selection means for reproducing, as a moving image, the image data captured in time series.

3. The image processing apparatus according to claim 2, wherein:
a detection method that can be selected by the detection method selection means is one of or a combination of at least two of:
a method by which the image data captured in time series is extracted at constant intervals;
a method by which image data is extracted on the basis of a difference in image data between an n-th image and an (n±m) th image (where n and m are arbitrary integers) that are successive in time series;
a method by which the image data is extracted on the basis of a characteristic of a lesion site;
a method by which image data is extracted on the basis of a color component of the image data; and
a method by which image data is extracted on the basis of a difference in image data between an n-th image (where n is an arbitrary integer) and an average value of p images (where p is an arbitrary integer) that are successive in time series before and after the n-th image.

4. The image processing apparatus according to claim 3, wherein:
the display control means is capable of displaying an image that was not extracted by the detection method.

5. The image processing apparatus according to claim 1, wherein:
the display control means displays a list of j vertical thumbnail images by k horizontal thumbnail images (j and k are arbitrary integers) of the image data.

6. The image processing apparatus according to claim 5, wherein:
setting of the j by k is performed arbitrarily by a user, or is determined on the basis of size of the thumbnail images.

7. The image processing apparatus according to claim 5, wherein:
the display control means further displays, in an emphasized manner, a thumbnail image including a lesion site, or assigns prescribed identification information to a thumbnail image including a lesion site among the thumbnail images in the displayed list.

8. The image processing apparatus according to claim 5, wherein:
when a prescribed thumbnail image is selected from among the thumbnail images in the displayed list, the display control means further displays at least one of:
a command of assigning remark information to image data corresponding to the selected thumbnail image;
a command of adding the selected thumbnail image to a prescribed image display area;
a command of attaching, to a report for a case, image data corresponding to the selected thumbnail image;
a command of indicating a position of image data corresponding to the selected thumbnail image on a time display axis displayed as a time scale representing an image capturing term of a series of pieces of the image data captured in time series;
a command of transitioning to a moving-image display screen for reproducing, as a moving image, image data after image data corresponding to the selected thumbnail image, and of reproducing the image data as a moving image;
a command of displaying x images (x is an arbitrary integer) before and after image data corresponding to the selected thumbnail image;
a command of displaying all images between the selected thumbnail image and a time-series thumbnail image immediately before the selected thumbnail image in the displayed list; and
a command of displaying all images between the selected thumbnail image and a time-series thumbnail image immediately after the selected thumbnail image displayed in the list.

9. The image processing apparatus according to claim 1, wherein:
the image detection means detects the prescribed image as the detection image data from image data captured in time series, by using at least one of:
an image detection routine of detecting one image for each prescribed time period;
an image detection routine of detecting adjacent images having a low similarity between them;
an image detection routine of detecting an image including a lesion site;
an image detection routine of detecting an image other than images unnecessary to diagnoses; and
an image detection routine of detecting an image including an image or an area that includes pixels of a prescribed color component at a high rate.

10. The image processing apparatus according to claim 9, wherein:
the display control means is capable of displaying an image that was not extracted by the image detection routine.

11. The image processing apparatus according to claim 9, wherein:
the image processing apparatus further comprises:
image detection routine selection means for selecting the image detection routine.

12. The image processing apparatus according to claim 11, wherein:
the image processing apparatus further comprises:
association means for associating the detection image data detected by the image detection means with an image detection routine name used for the detection; and
the display control means displays the image detection routine name as a label name together with the images in the displayed list.

13. The image processing apparatus according to claim 9, wherein:
the image processing apparatus further comprises:
remark information assignment means for assigning remark information to an image selected from among the images in the displayed list.

14. The image processing apparatus according to claim 13, wherein:
the image processing apparatus further comprises:
report preparation means for preparing a report on a case relating to an image selected from among the images in the displayed list; and
remark information insertion means for inserting remark information into the report together with the selected image when the remark information has been assigned to the selected image.

15. The image processing apparatus according to claim 14, wherein:
the image processing apparatus further comprises:
lump output means for outputting, en masse, all detection image data detected by the image detection means to the report.

16. The image processing apparatus according to claim 1, wherein:
the display control means displays a list of detection image data, which is the image data that was detected, and displays abstract information of all images captured in time series; and further assigns, to the abstract information, indication information for indicating a position of the selected image in the abstract information so as to display the indication information in connection with an operation of selecting an arbitrary image from among the detection images in the displayed list.

17. The image processing apparatus according to claim 16, wherein:
the display control means assigns, to the abstract information, indication information indicating a section of an image group in the displayed list within an image group included in the abstract information, and displays the indication information.

18. The image processing apparatus according to claim 16, wherein:
the display control means scrolls the detection images in units of liens, pages or images when a list of the detection images is displayed.

19. The image processing apparatus according to claim 16, wherein:
when a moving-image display screen for displaying, as a moving image, the images captured in time series transitions to a list display screen for displaying a list of the detection images, the display control means
displays a list from a top of the detected time-series detection image group,
displays a list of the detection images before and after an image being displayed on the moving-image display screen,
displays a list of the detection images in a range including an image being displayed on the moving-image display screen,
displays, at a top of a page of a list of the detection images, an image being displayed on the moving-image display screen, or
displays, at a bottom of a page of a list of the detection images, an image being displayed on the moving-image display screen.

20. The image processing apparatus according to claim 19, wherein:
when a moving-image display screen for displaying, as a moving image, the images captured in time series transitions to a list display screen for displaying a list of the detection images, positions of the images displayed on the list display screen can be set.

21. The image processing apparatus according to claim 16, wherein:
when a list display screen for displaying a list of the detection images transitions to a moving-image display screen for displaying, as a moving image, the images captured in time series, the display control means displays a first image among the images captured in time series, displays again an image displayed on the moving-image display screen before transitioning to the list display screen, or displays an image selected on the list display screen.

22. The image processing apparatus according to claim 16, wherein:
the display control means assigns, to the abstract information, indication information for indicating a page section, which is a unit of an image group included in a displayed list at one time, and displays the indication information.

23. The image processing apparatus according to claim 16, wherein:
the display control means assigns, to the image selected from among the images in the displayed list, identification information indicating the selection, and displays the identification information by using a mark or displays the identification information in an emphasized manner, or adds the selected image to a sub-display area for displaying only the selected image so as to display the selected image.

24. The image processing apparatus according to claim 23, wherein:
the display control means scrolls the image displayed in the sub-display area in connection with scrolling of a list display screen, and also displays only an image in the section in the list displayed in the sub-display area.

25. The image processing apparatus according to claim 16, wherein:
the display control means further adds an image arbitrarily selected on a moving-image display screen for displaying, as a moving image, the images captured in time series to a list display screen for displaying a list of the detection images so as to display the selected image, and displays, in an emphasized manner, the image selected arbitrarily from the images detected and included in the displayed list, or assigns identification information for identifying the selected image so as to display the identification information.

26. The image processing apparatus according to claim 16, wherein:
the display control means assigns, to the abstract information, identification information for indicating positions of all the detected image data in the abstract information.

27. The image processing apparatus according to claim 26, wherein:
the display control means further displays a list including only images corresponding to the identification information included in a desired range in the abstract information, and displays the abstract information in the desired range in an enlarged manner.

28. The image processing apparatus according to claim 16, wherein:
the detection images in the displayed list can be specified in units of lines or pages.

29. The image processing apparatus according to claim 1, wherein:
the display control means displays, in a first direction, detection image data detected by the image detection means from the time-series image data, and displays, in a second direction from the detection image data, at least zero pieces of the time-series image data corresponding to frame images before and after each detection image data.

30. The image processing apparatus according to claim 1, wherein:
when a cursor is on each of the images in the displayed list, the display control means displays that image in an enlarged manner.

31. The image processing apparatus according to claim 3, wherein:
the image processing apparatus further comprises:
similarity threshold value setting means that is capable of adjusting a level of similarity between images to be detected, by setting a threshold value of similarity between pieces of the image data.

32. The image processing apparatus according to claim 1, wherein:
the image processing apparatus further comprises:
section setting means for specifying a time-series image in an arbitrary section from the time-series image data; and
the display control means displays a list of a time-series image group in the specified section.

33. The image processing apparatus according to claim 31, wherein:
the image processing apparatus further comprises:
section setting means for setting a time-series image in an arbitrary section from the time-series image data; and
the display control means displays a list of detection image data from time-series images in the specified section on the basis of the threshold value.

34. The image processing apparatus according to claim 1, wherein:
the image processing apparatus further comprises:
setting means for specifying a plurality of arbitrary sections in the time-series image data, and setting a threshold value for detecting, by using the image detection means, images for each of the specified sections.

35. The image processing apparatus according to claim 1, wherein:
when the image groups are included in a displayed list ranging over a plurality of pages, the display control means turns pages each time a prescribed time period has elapsed.

36. The image processing apparatus according to claim 3, wherein:
the image display means further comprises:
reproducing time period setting means for setting a time period for reproducing the detection image, and
the display control means calculates a number of images to be reproduced from a frame rate on the basis of the set time period, and determines a threshold value of the difference in image data so that the detection image data for the calculated number of images can be obtained.

37. An image processing apparatus, comprising:
obtainment means for obtaining time-series image data, which is image data captured in time series by a capsule endoscope;
image detection means for detecting prescribed image data from the obtained time-series image data;
display means including a main display area for displaying an image, and two sub-display areas that are smaller than the main display area and that are for displaying an image;
display control means for reproducing, in the main display area, detection image data detected from the time-series image data by the image detection means, reproducing and displaying detection image data before and after the detection image data in each of the sub-display areas, displaying, when the reproduction is stopped, detection image data at a moment of the stoppage in the main display area, and displaying the time-series image data corresponding to frame images before and after the detection image data in the sub-display areas.

38. A method of operating an image processing apparatus, comprising:
obtaining time-series image data, which is image data captured in time series by a capsule endoscope;
detecting prescribed image data from the obtained time-series image data; and
displaying a list of detection image data, which is the detected image data.

39. The method of operating an image processing apparatus according to claim 38, comprising:
selecting a detection method used when the detection is performed on the basis of an instruction input to a display instruction input screen to which an instruction about displaying is input;
selecting a listing display for performing the listing display; or
reproducing, as a moving image, the image data captured in time series.

40. The method of operating an image processing apparatus according to claim 39, wherein:
a detection method that can be selected in the display instruction input screen is one of or a combination of at least two of:
a method by which the image data captured in time series is extracted at constant intervals;
a method by which image data is extracted on the basis of a difference in image data between an n-th image and an (n±m) th image (where n and m are arbitrary integers) that are successive in time series;
a method by which the image data is extracted on the basis of a characteristic of a lesion site;
a method by which image data is extracted on the basis of a color component of the image data; and
a method by which image data is extracted on the basis of a difference in image data between an n-th image (where n is an arbitrary integer) and an average value of p images (where p is an arbitrary integer) that are successive in time series before and after the n-th image.

41. The method of operating an image processing apparatus according to claim 40, further comprising:
displaying an image that was not extracted by the detection method.

42. The method of operating an image processing apparatus according to claim 38, comprising:
displaying a list of j vertical thumbnail images by k horizontal thumbnail images (j and k are arbitrary integers) of the image data.

43. The method of operating an image processing apparatus according to claim 42, wherein:
setting of the j by k is performed arbitrarily by a user, or is determined on the basis of size of the thumbnail images.

44. The method of operating an image processing apparatus according to claim 42, further comprising:
displaying, in an emphasized manner, a thumbnail image including a lesion site, or assigning prescribed identification information to a thumbnail image including a lesion site among the thumbnail images in the displayed list.

45. The method of operating an image processing apparatus according to claim 42, comprising, when a prescribed thumbnail image is selected from among the thumbnail images in the displayed list, displaying at least one of:
a command of assigning remark information to image data corresponding to the selected thumbnail image;
a command of adding the selected thumbnail image to a prescribed image display area;
a command of attaching, to a report for a case, image data corresponding to the selected thumbnail image;
a command of indicating a position of image data corresponding to the selected thumbnail image on a time display axis displayed as a time scale representing an image capturing term of a series of pieces of the image data captured in time series;
a command of transitioning to a moving-image display screen for reproducing, as a moving image, image data after image data corresponding to the selected thumbnail image, and of reproducing the image data as a moving image;
a command of displaying x images (x is an arbitrary integer) before and after image data corresponding to the selected thumbnail image;
a command of displaying all images between the selected thumbnail image and a time-series thumbnail image immediately before the selected thumbnail image in the displayed list; and
a command of displaying all images between the selected thumbnail image and a time-series thumbnail image immediately after the selected thumbnail image displayed in the list.

46. The method of operating an image processing apparatus according to claim 38, comprising detecting the prescribed image as the detection image data from the image data captured in time series, by using at least one of:
an image detection routine of detecting one image for each prescribed time period;
an image detection routine of detecting adjacent images having a low similarity between them;
an image detection routine of detecting an image including a lesion site;
an image detection routine of detecting an image other than images unnecessary to diagnoses; and
an image detection routine of detecting an image including an image or an area that includes pixels of a prescribed color component at a high rate.

47. The method of operating an image processing apparatus according to claim 46, comprising:
displaying an image that was not extracted by the image detection routine.

48. The method of operating an image processing apparatus according to claim 46, further comprising:
enabling selection of the image detection routine.

49. The method of operating an image processing apparatus according to claim 46, further comprising:
associating the detection image data, which is the detected image data, with an image detection routine name used for the detection; and
displaying the image detection routine name as a label name together with the images in the displayed list.

50. The method of operating an image processing apparatus according to claim 46, further comprising:
assigning remark information to an image selected from among the images in the displayed list.

51. The method of operating an image processing apparatus according to claim 50, further comprising:
preparing a report on a case relating to an image selected from among the images in the displayed list; and
inserting the remark information into the report together with the selected image when the remark information has been assigned to the selected image.

52. The method of operating an image processing apparatus according to claim 51, further comprising:
outputting, en masse, all detected detection images to the report.

53. The method of operating an image processing apparatus according to claim 38, comprising:
displaying a list of detection image data, which is the image data that was detected, and displaying abstract information of all images captured in time series; and further assigning, to the abstract information, indication information for indicating a position of the selected image in the abstract information so as to display the indication information in connection with an operation of selecting an arbitrary image from among the detection images in the displayed list.

54. The method of operating an image processing apparatus according to claim 53, comprising:
assigning, to the abstract information, indication information indicating a section of an image group in the displayed list among an image group included in the abstract information, and displaying the indication information.

55. The method of operating an image processing apparatus according to claim 53, comprising:
scrolling the detection images in units of liens, pages or images when a list of the detection images is displayed.

56. The method of operating an image processing apparatus according to claim 53, comprising, when a moving-image display screen for displaying, as a moving image, the images captured in time series transitions to a list display screen for displaying a list of the detection images:
displaying a list from a top of the detected time-series detection image group,
displaying a list of the detection images before and after an image being displayed on the moving-image display screen,
displaying a list of the detection images in a range including an image being displayed on the moving-image display screen,
displaying, at a top of a page of a list of the detection images, an image being displayed on the moving-image display screen, or
displaying, at a bottom of a page of a list of the detection images, an image being displayed on the moving-image display screen.

57. The method of operating an image processing apparatus according to claim 13, wherein:
when a moving-image display screen for displaying, as a moving image, the images captured in time series transitions to a list display screen for displaying a list of the detection images, positions of the images displayed on the list display screen can be set.

58. The method of operating an image processing apparatus according to claim 53, comprising:
displaying a first image among the images captured in time series, displaying again an image displayed on the moving-image display screen before transitioning to the list display screen, or displaying an image selected on the list display screen, when a list display screen for displaying a list of the detection images transitions to a moving-image display screen for displaying, as a moving image, the images captured in time series.

59. The method of operating an image processing apparatus according to claim 53, comprising:
assigning, to the abstract information, indication information for indicating a page section, which is a unit of an image group included in a displayed list at one time, and displaying the indication information.

60. The method of operating an image processing apparatus according to claim 53, comprising:
assigning, to the image selected among the images in the displayed list, identification information indicating the selection, and displaying the identification information by using a mark or displaying the identification information in an emphasized manner, or adding the selected image to a sub-display area for displaying only the selected image so as to display the selected image.

61. The method of operating an image processing apparatus according to claim 60, comprising:
scrolling the image displayed in the sub-display area in connection with scrolling of a list display screen, and also displaying only an image in the section in the list displayed in the sub-display area.

62. The method of operating an image processing apparatus according to claim 53, further comprising
adding an image arbitrarily selected on a moving-image display screen for displaying, as a moving image, the images captured in time series to a list display screen for displaying a list of the detection images so as to display the selected image, and displaying, in an emphasized manner, the image selected arbitrarily from the images detected and included in the displayed list, or assigning identification information for identifying the selected image so as to display the identification information.

63. The method of operating an image processing apparatus according to claim 53, comprising:
assigning, to the abstract information, identification information for indicating positions of all the detected image data in the abstract information.

64. The method of operating an image processing apparatus according to claim 63, further comprising:
displaying a list including only images corresponding to the identification information included in a desired range in the abstract information, and displaying the abstract information in the desired range in an enlarged manner.

65. The method of operating an image processing apparatus according to claim 53, wherein:
the detection images in the displayed list can be specified in units of lines or pages.

66. The method of operating an image processing apparatus according to claim 38, comprising:
displaying, in a first direction, detection image data detected by the image detection means from the time-series image data, and displaying, in a second direction from the detection image data, at least zero piece of the time-series image data corresponding to frame images before and after each detection image data.

67. The method of operating an image processing apparatus according to claim 38, comprising:
displaying, when a cursor is on each of the images in the displayed list, that image in an enlarged manner.

68. The method of operating an image processing apparatus according to claim 40, further comprising:
adjusting a level of similarity between images to be detected by setting a threshold value about similarity between pieces of the image data.

69. The method of operating an image processing apparatus according to claim 38, further comprising:
specifying a time-series image in an arbitrary section from the time-series image data; and
displaying a list of a time-series image group in the specified section.

70. The method of operating an image processing apparatus according to claim 68, comprising:
specifying a time-series image in an arbitrary section from the time-series image data; and
displays a list of detection image data from time-series images in the specified section on the basis of the threshold value.

71. The method of operating an image processing apparatus according to claim 38, further comprising:
specifying a plurality of arbitrary sections in the time-series image data, and setting a threshold value for detecting images for each of the specified sections.

72. The method of operating an image processing apparatus according to claim 38, comprising:
turning pages each time a prescribed time period has elapsed when the image groups are included in a displayed list ranging over a plurality of pages.

73. The method of operating an image processing apparatus according to claim 40, comprising:
setting a time period for reproducing the detection image, and
calculating a number of images to be reproduced from a frame rate on the basis of the set time period, and determining a threshold value of the difference in image data so that the detection image data for the calculated number of images can be obtained.

74. A method of operating an image processing apparatus, comprising:
obtaining time-series image data, which is image data captured in time series by a capsule endoscope;
detecting prescribed image data from the obtained time-series image data;
displaying a main display area for displaying an image, and two sub-display areas that are smaller than the main display area and that are for displaying an image;
reproducing, in the main display area, detection image data detected from the time-series image data, reproducing and displaying detection image data before and after the detection image data in each of the sub-display areas, displaying, when the reproduction is stopped, detection image data at a moment of the stoppage in the main display area, and displaying the time-series image data corresponding to frame images before and after the detection image data in the sub-display areas.

75. An image display program for making a computer perform a process of displaying an image, said program making the computer execute:
an obtainment step of obtaining time-series image data, which is image data captured in time series by a capsule endoscope;
an image detection step of detecting prescribed image data from the obtained time-series image data; and
a display control step of displaying a list of detection image data, which is the detected image data.
